# EUROPEAN PATENT APPLICATION

(11) **EP 1 604 569 A1**
(43) Date of publication of application: **14.12.2005**
(21) Application number: 04712724.6
(22) Date of filing: 19.02.2004
(51) Int. Cl.: A01M 1/02, A01M 1/20, A01M 7/00

(54) **CHEMICAL DIFFUSION SYSTEM, CHEMICAL DIFFUSION APPARATUS, CHEMICAL DIFFUSION UNIT AND CHEMICAL CARTILAGE**

(30) Priority: 14.03.2003 JP 2003070544; 18.08.2003 JP 2003294680; 18.08.2003 JP 2003294679
(71) Applicant: SEIKO EPSON CORPORATION, Shinjuku-ku, Tokyo 163-0811 (JP)
(72) Inventor: Ito, Kiyoshi, c/o Seiko Epson Corporation, Suwa-shi, Nagano 3928502 (JP); Yamada, Manabu, c/o Seiko Epson Corporation, Suwa-shi, Nagano 3928502 (JP)
(74) Representative: Hoffmann, Eckart
(86) International application number: PCT/JP2004/001940
(87) International publication number: WO 2004/080169

(57) **Abstract**

A chemical diffusion apparatus (1) comprises a first tank (2) filled up with a volatile chemical A such as a communication disturbance agent and a second tank (3) filled up with a volatile chemical B. The chemicals A and B are sucked by means of a discharge volume variable pump (6) to be discharged onto an evaporating dish (8) through a discharge tube (7). A mixed chemical C discharged onto the evaporating dish 8 is naturally diffused in the air. A control circuit (9) controls drive of the pump (6). An input side of the control circuit (9) is connected to a timer (11) and an environment parameter detecting means such as a temperature sensor (12), an illumination sensor (13), a wind direction sensor (14) and a wind velocity sensor (15). The control circuit (9) controls discharge and discharge volume of the mixed chemical C from the pump (6) on the basis of detecting results from the respective sensors in accordance with a control program.

## Description

### Technical Field

The invention relates to a chemical diffusion system, a chemical diffusion apparatus, a chemical diffusion unit and a chemical cartridge, which are used for diffusing chemicals such as a communication disturbance agent and an attractant for a noxious insect, the chemicals including an insect sex pheromone.

### Related Art

There has been a noxious insects extermination method known such that an attractant such as an insect sex pheromone is diffused in the air to cause a communication disturbance of insects for reducing the ovipositional number, and thereby, reducing growth of noxious insects in a farm or the like instead of using an insecticide. For example, known is a method in which a large number of insect sex pheromone dispensers are installed within a predetermined space in cultivation field facilities to naturally diffuse an insect sex pheromone. In such a method, a sex pheromone is put in a plastic tube, which is hooked on a branch of a plant under cultivation or a frame of a plastic greenhouse and through which the sex pheromone is naturally diffused in the air, for example (JP-A-8-322447).

In the method for naturally diffusing a chemical such as a sex pheromone included in the plastic tube, however, diffusion is continuously carried out even in the daytime when insects do not mate. This causes much unnecessary diffusion of a chemical since the diffusion amount is more in the daytime when the temperature is high. Accordingly, a chemical such as a sex pheromone cannot be effectively diffused in the method of natural diffusion. Further, the amount of diffusion of a chemical should be adjusted in accordance with the number of installed dispensers. This causes a problem that an installing operation becomes time-consuming since a large number of dispensers should be installed generally. Moreover, different kinds of dispensers should be installed in order to exterminate different kinds of noxious insects since a kind and constituents of a chemical diffused from the dispenser are determined in advance. In addition, there is a problem that a conventional natural diffusion type dispenser of the throwaway-type, and thus, uneconomical.

On the other hand, there have been chemical diffusion apparatuses proposed for forcibly diffusing a volatile chemical by means of a heater or a fan (JP-UM-A-58-110288, Japanese Utility Model Registration No. 3021119, JP-A-9-74969 and JP-A-2002-291392). Further, a remote-controlled spray type chemical diffusion apparatus has been proposed (US Patent No. 6,182,904). Using such forcible type chemical diffusion apparatuses allows the problems in using the above natural diffusion type dispenser to be solved.

A chemical diffusing operation by means of the conventional chemical diffusion apparatuses, however, is of a fixed type such that a predetermined amount of chemical is diffused in a predetermined time period or zone. It is known that a chemical such as insect sex pheromone is not effective unless it is diffused in the optimum time period or zone in accordance with a parameter related to an ecological system of a noxious insect subject to extermination or an ambient environment parameter. In an example of a parameter of an ecological system, for example, it is known that there are several peaks of mating of subject noxious insects after a predetermined time period after sunset for each kind of noxious insects. That is to say, starting time and ending time for chemical diffusion should be changed since the time of sunset is different in accordance with latitude, longitude and the seasons.

Furthermore, because the hours of sunlight can vary based upon weather as determined by an artificial weather controlling apparatus, it is also required to make a change from the natural sunset time. Additionally, it is known that other environmental parameter such as a wind velocity, temperature and illumination also influences an activity of insects.

The temperature is a parameter relating to an ecological system of insects as well as an environment parameter influencing evaporation of a chemical. As for a wind velocity and a wind direction, a chemical must be diffused much more in the case of installing a chemical diffusing apparatus on the upwind side than the case of installing the apparatus on the downwind side. Accordingly, the conventional chemical diffusion apparatus cannot optimally diffuse a chemical in accordance with a pattern of behavior of a noxious insect subject to extermination, weather, or a place of installing the chemical diffusion apparatus. Moreover, the conventional chemical diffusion apparatus is required to change a control mode of a chemical diffusing operation on the basis of the optimum diffusion condition at whenever the place of installation is changed. Similarly, in the case of changing a kind of a chemical, the control mode should be changed so as to correspond to a diffusion condition optimum to the changed chemical. When a diffusing operation is carried out through computer control, for example, it is required to rewrite a control program for chemical diffusion in accordance with a change of a place of installation or a chemical. Accordingly, a change in place of installation and chemical cannot be easily performed.

In addition, when different kinds of chemicals are diffused by means of a same chemical diffusion apparatus, not only cleaning or a change of an evaporating dish, a porous material or a fibriform material, which is used as a diffusing means, is required, but also cleaning of a part such as a chemical supply path for supplying a chemical from a chemical supply source to the diffusing means is necessary. From such a point of view, it is also known that changing a chemical is not easy with the conventional chemical diffusion apparatus.

On the other hand, a chemical such as an insect sex pheromone is easily affected by heat typically, and therefore, a method of heating an evaporating dish by means of a heater to forcibly diffuse a chemical cannot be used in many cases. In such a case, a porous material or a fibriform material, which has a large surface area, is generally used as a diffusing means in order to naturally diffuse or transpire a chemical with high efficiency. In the case of using a porous material or a fibriform material, however, dirt and dust are caught thereon, and thereby, easily cause clogging. Accordingly, the porous or fibriform material should be regularly cleaned or changed for the purpose of preventing deterioration in diffusion efficiency. Such a maintenance operation is extremely troublesome when the chemical diffusion apparatus is installed in a high place.

Furthermore, in the case of a spray-type chemical diffusion apparatus, reusing (refilling) a used spray can is difficult. That is to say, refilling a spray can requires cleaning of the inside thereof, but such cleaning is not easy to carry out. This causes difficulty in reusing a spray can. Moreover, when a chemical such as an insect sex pheromone is diffused, mixing affinity for pressurized gas for spraying such as LPG and butane is necessary to be checked. There are, however, not many kinds of insect sex pheromone for which the mixing affinity has been verified. Therefore, a conventional spray type chemical diffusion apparatus cannot be widely used.

### Disclosure of the Invention

A main object of the invention is to provide a chemical diffusion system, a chemical diffusion apparatus, a chemical diffusion unit and a chemical cartridge, which are capable of diffusing a chemical such as an insect sex pheromone in the optimum time period or zone in accordance with a pattern of behavior of a noxious insect subject to extermination or an environment.

Another main object of the invention is to provide a chemical diffusion system, a chemical diffusion apparatus, a chemical diffusion unit and a chemical cartridge, which are capable of easily corresponding to a change in place of installation.

Further another main object of the invention is to provide a chemical diffusion system, a chemical diffusion apparatus, a chemical diffusion unit and a chemical cartridge, which are capable of easily corresponding to a change in chemical.

For the purpose of achieving the above main objects and other objects, a chemical diffusion apparatus in accordance with the invention is a chemical diffusion apparatus comprising: at least one chemical tank for reserving a chemical such as an insect sex pheromone; a discharging means for discharging the chemical from the chemical tank; and a diffusing means for diffusing the chemical discharged from the discharging means, wherein the controlling means carries out drive control by varying diffusion timing and/or discharge volume of the diffusing means on the basis of hours of sunlight or sunset time, which are varied in accordance with a season, latitude and longitude and control by an artificial weather controlling apparatus.

In the invention, discharge timing and/or discharge volume are changed on the basis of hours of sunlight or sunset time. Accordingly, the optimum amount of chemical can be discharged in a time period or zone in which efficiency is highest.

Further, a chemical diffusion apparatus in accordance with the invention comprises: an environment parameter detecting means; and a controlling means for controlling drive of the discharging means on the basis of a detection result of the environment parameter detecting means, wherein the environment parameter detecting means detects at least one environment parameter of a temperature, humidity, illumination, a wind direction, a wind velocity and a chemical concentration.

In the invention, the environment parameter detecting means detects a temperature, a wind direction and the like, and then, the controlling means controls the discharging means on the basis of the above detection to discharge a chemical. Accordingly, a chemical can be diffused so as to be most effective for attraction in accordance with a behavior pattern of a subject noxious insect in the case of diffusing a communication disturbance agent for an insect sex pheromone or the like. This allows unnecessary chemical diffusion to be prevented, so that the maximum effect can be achieved with the least amount of chemical.

In the above, arranging the chemical diffusion apparatus to comprise a time detecting means such as a timer or a radio timepiece and the controlling means to control drive of the discharging means on the basis of the time detected by means of the time detecting means allows a time period or zone in which a noxious insect subject to extermination is active such as a time period or zone after sunset to be accurately detected.

The chemical diffusion apparatus in accordance with the invention may be arranged to include a self-information memorizing means for holding at least information relating to a place of installation and the controlling means control drive of the discharging means operates on the basis of the information held in the self-information memorizing means. In the case of a chemical diffusion apparatus installed at a corner of a chemical diffusion place, which is the downwind side, for example, a chemical diffused on the upstream side flows thereto, and thus, little discharge volume is required. Contrary to the above, the discharged chemical flows to the downwind side when the chemical diffusion apparatus is installed on the upwind side, and therefore, the discharge amount of the chemical should be more than the usual case. The controlling means can carry out such control on the basis of location information held in the self-information memorizing means. As a result of the above, a chemical can be diffused evenly all over the subject area.

In the chemical diffusion apparatus according to the invention, it may be arranged that the chemical tank include plural tanks containing respective chemical constituents that can be discharged from the respective tanks by means of the discharging means and mixed to form the various chemicals. It may also be arranged that the chemical tank include plural tanks reserving different chemicals and the discharging means be able to discharge chemicals from the respective tanks individually or simultaneously. In the case that a noxious insect subject to extermination is changed, for example, changing a mixing rate of the chemical constituents can correspond to the new insect. Also, discharging a different chemical from a tank different from a tank used up to that time can correspond to the new insect.

Moreover, a chemical tank in accordance with the invention is preferably formed from a material capable of cutting off ultraviolet rays and/or oxygen. This allows oxidation and/or ultraviolet-rays deterioration of a chemical such as an insect sex pheromone to be prevented, so that an antioxidant or an ultraviolet-rays stabilizer are not required to be mixed with the chemical.

As the discharging means, preferably used is one variable in discharge volume of a chemical. It is desirable to use a pump variable in discharge volume, for example. It is also possible to use one having a structure similar to that of an inkjet head of an inkjet printer instead of the above. In this case, the discharging means includes a chemical pressure chamber supplied with the chemical from the chemical tank, a chemical discharge nozzle communicating with the chemical pressure chamber and an actuator for generating variations in pressure of the chemical in the chemical pressure chamber to discharge liquid drops of the chemical from the chemical discharge nozzle.

Further, an evaporating dish for evaporating the chemical or a chemical holding member formed from a porous material or a fibriform material for holding the chemical so as to be capable of natural diffusion may be used as the diffusing means.

In the case of diffusing a very small amount of chemical in a wide area or diffusing a chemical having a low coefficient of diffusion (a chemical difficult to be diffused), it is desirable to use a chemical diffusion apparatus arranged to include as the diffusing means a chemical holding member for receiving the chemical discharged from the discharging means to hold the chemical so as to be capable of natural diffusion and a carrying mechanism for circulating the chemical holding member along a predetermined carrying path. Such arrangement allows an operation of installing a large number of chemical diffusion apparatuses to be unnecessary.

Moreover, a changeable chemical cartridge may be used as the chemical tank. In this case, the chemical diffusion unit using the chemical cartridge as a chemical supply source includes a cartridge mounting part for changeably mounting the chemical cartridge, the discharging means, the diffusing means, the environment parameter detecting means and the controlling means.

Additionally, a chemical diffusion apparatus according to the invention comprises: a chemical cartridge used as the chemical tank; and a chemical diffusion unit using the chemical cartridge as a chemical supply source, wherein the chemical cartridge includes a chemical reservoir part for reserving the chemical and a cartridge side memorizing means for memorizing at least one controlling parameter relating to the chemical diffusing operation, the chemical diffusion unit includes a cartridge mounting part for changeably mounting the chemical cartridge, the discharging means, the diffusing means, the environment parameter detecting means and the controlling means, and the controlling means controls drive of the discharging means on the basis of the controlling parameter memorized in the cartridge side memorizing means of the chemical cartridge mounted to the cartridge mounting part.

In the invention, a chemical cartridge changeably mounted to the chemical diffusion unit is used as a chemical supply source and the chemical cartridge is arranged to hold a controlling parameter for controlling the chemical diffusing operation. Accordingly, only changing the chemical cartridge is enough in order to diffuse a different chemical. When a new chemical cartridge is mounted, a system capable of executing a chemical diffusing operation satisfying the optimum chemical diffusion condition can be constructed in the chemical diffusion unit on the basis of the controlling parameter held in the new chemical cartridge. Thus, holding more controlling parameters in the chemical cartridge allows a change of a setting condition of the diffusing operation of the chemical diffusing unit to be made unnecessary or simplified in changing a chemical or changing a place of installing the chemical diffusion unit.

It is possible in the above to hold all controlling parameters relating to chemical diffusion in the chemical cartridge while controlling parameters not affected by the kind of the chemical cartridge can be held in the chemical diffusion unit. That is to say, it may be arranged that the chemical diffusion unit include a unit side memorizing means for memorizing at least one controlling parameter relating to the chemical diffusing operation, the controlling parameter being different from the controlling parameter held in the chemical cartridge, and the controlling means control drive of the discharging means on the basis of the controlling parameter of the chemical cartridge and the controlling parameter of the chemical diffusion unit.

Typical controlling parameters include the following:
(a) a kind of the chemical;
(b) volume and a remaining amount of the chemical;
(c) a kind of a noxious insect subject to extermination by means of the chemical;
(d) the optimum condition for diffusing the chemical;
(e) location information of the chemical diffusion unit; and
(f) chemical discharge power of the chemical diffusion unit.

In a typical structure, the controlling parameter held in the chemical cartridge includes at least the optimum condition of diffusing the chemical, and the controlling parameter held in the chemical diffusion unit includes at least location information of the chemical diffusion unit.

Further, it may be arranged that a drive controlling means of the chemical diffusion unit be formed from a computer, the chemical cartridge hold a drive controlling program of the drive controlling means, and the chemical diffusion unit read the drive controlling program when the chemical cartridge is mounted, and then, the drive controlling program be executed to perform an operation of diffusing the chemical. This allows the chemical diffusion unit to be standardized with the minimum function. Moreover, it is enough to only change the chemical cartridge without changing the driving program in the chemical diffusion unit when a chemical is changed.

In this case, the drive controlling program can be read from the chemical cartridge having the first priority determined on the basis of the order of priority of the chemical cartridge, the order of priority of the cartridge mounting part of the chemical diffusion unit or the order of mounting the chemical cartridge when the chemical diffusion unit uses the plural chemical cartridges as a chemical supply source.

In order to form the chemical diffusion unit with the minimum elements, it is desirable that a battery power source is mounted to the chemical cartridge and the chemical cartridge is mounted to the chemical diffusion unit to supply the chemical diffusion unit with driving power from the battery power source.

On the other hand, it may be arranged that the chemical diffusion unit hold unit identification information for identifying the chemical diffusion unit and information of customers purchasing the chemical diffusion unit and that the unit identification information and the purchaser information be held in the chemical cartridge by means of the chemical diffusion unit when the chemical cartridge is mounted.

This allows the unit identification information and the purchaser information, which are held in the used chemical cartridge, to be read to construct a database relating to purchasers. Meticulous service such as an offer of chemical information to the purchasers can be offered on the basis of the constructed database. For the purpose of the above, a writing apparatus for writing at least one of the control parameters, the drive controlling program or the purchaser information and a database construction apparatus for reading the unit identification information and the purchaser information, which are held in the used chemical cartridge, to construct a database relating to the purchasers are included as the cartridge side memorizing means.

Moreover, the chemical diffusion apparatus according to the invention comprises: a chemical cartridge used as the chemical tank; and a chemical diffusion unit using the chemical cartridge as a chemical supply source, wherein the chemical cartridge includes a chemical reservoir part for reserving the chemical, the diffusing means and/or the chemical supply tube for connecting the chemical reservoir part with the diffusion means and the chemical diffusion unit includes at least a cartridge mounting part for changeably mounting the chemical cartridge, the environment parameter detecting means and the controlling means.

In the invention, a chemical cartridge changeably mounted to the apparatus is used as a chemical supply source of the chemical diffusion unit. When the diffusing means is mounted to the chemical cartridge, changing the used chemical cartridge allows the diffusing means to be changed simultaneously. Accordingly, using the porous material or a fibriform material for the diffusing means basically requires no maintenance thereof since the chemical cartridge is changed together with the diffusing means. In the case that a chemical supply tube is mounted to a chemical cartridge, cleaning of the chemical diffusion unit is unnecessary when a kind of a chemical is changed since no chemical supply path is provided in the chemical diffusion apparatus. In addition, in the case that a diffusing means and a chemical supply tube are mounted to a chemical cartridge, the chemicals do not contact with a component of the chemical diffusion unit. Accordingly, only changing the chemical cartridge into a necessary one is required when a kind of a chemical is changed.

In the case of including a chemical supplying tube, it is desirable to use as the discharging means a chemical supply pump for supplying the diffusing means with the chemical in the chemical reservoir part and to use as the chemical supply pump a tube pump including the chemical supply tube and a driving part for pressuring the chemical supply tube to send out the chemical in the chemical tube. In this case, the driving part can be mounted to the chemical diffusion unit.

It is also possible to arrange that a pressure mechanism for pressuring the chemical reserved in the chemical reservoir part and a valve mechanism for opening and closing the chemical supply tube be included and the pressure mechanism, and the valve mechanism be mounted to the chemical diffusion unit. Controlling the valve mechanism allows the chemical under pressure to be pushed out from the chemical reservoir part to be supplied to the diffusing means when the chemical supply tube is opened according to the necessity.

As the diffusing means, a member formed from a porous material or a fibriform material may be used. Mounting such a member to the chemical cartridge basically requires no maintenance thereof since the member is changed every time when the chemical cartridge is changed, as described above.

Further, arranging the chemical cartridge to include a chemical spray mechanism for diffusing a chemical in the air by means of pressurized gas allows the chemical spray mechanism to function as the discharging means and the diffusing means.

In this case, the chemical spray mechanism may include a pressurized gas reservoir part and the pressurized gas nozzle and an opening/closing mechanism for opening and closing the pressurized gas nozzle may be mounted to the chemical diffusing unit. That is to say, a chemical is reserved in the chemical cartridge while pressurized gas is reserved in the pressurized gas reservoir part differently from the conventional case of filling up a spray can with the chemical and the pressurized gas. When both of the above are separately reserved, it is not necessary to clean the inside to wash the chemical away in refilling the spray reservoir part filled up with pressurized gas such as a spray can, for example. In addition, it is not necessary to check affinity when the chemical and the pressurized gas are mixed for a reserve. Accordingly, a spray type diffusing means can be used in diffusion of various kinds of chemicals.

Moreover, changeably mounting at least one of the chemical reservoir part, the diffusing means, the chemical supply tube and the pressurized gas reservoir part to the chemical cartridge allows them to be individually changed.

In order to diffuse a chemical in a target place or in a wide area with one or a few chemical diffusion apparatuses, the chemical diffusion apparatus is mounted to a traveling mechanism to control drive of the traveling mechanism by means of the travel controlling means. Using such a mobile chemical diffusion system allows a chemical to be diffused in a wide area with high efficiency.

The chemical diffusion apparatus having the above structure may be hung on a balloon to float in a chemical diffusion place instead of using the traveling type chemical diffusion system. Such a floating type chemical diffusion system can be used in a greenhouse or the like and can be moved by means of a blower, for example.

In order to install plural chemical diffusion apparatuses, performing centralized control over the respective chemical diffusion apparatuses at one place allows a chemical to be properly diffused all over the subject area. A chemical diffusion system for the purpose of the above comprises: plural chemical diffusion apparatuses having the above structure and provided in different places; and a center controlling means for controlling respective chemical discharging operations of the chemical diffusion apparatuses, wherein the center controlling means controls discharge and/or discharge volume of the chemicals in the respective chemical diffusion apparatuses on the basis of a place for installing the respective diffusion apparatuses and a detection result by the environment parameter detecting means of the respective chemical diffusion apparatuses.

On the other hand, the invention relates to a chemical cartridge and a chemical diffusion unit, which respectively include the above structure.

### Brief Description of the Drawings

Figs. 1(a) and 1(b) are schematic views showing a structure of a chemical diffusion apparatus in accordance with Embodiment 1 of the invention and an illustration of a structure of a chemical tank thereof.
Fig. 2 is a schematic view showing a structure of a chemical diffusion apparatus in accordance with Embodiment 2 of the invention.
Figs. 3(a) and 3(b) are a schematic views showing a structure of a chemical diffusion system to which the invention is applied and an illustration showing a condition of installing the system.
Figs. 4(a) and 4(b) are a schematic views showing a structure of a chemical diffusion apparatus in accordance with Embodiment 3 of the invention and an illustration showing an example of arrangement of a belt conveyer thereof.
Figs. 5(a) and 5(b) are a schematic views showing a structure of a chemical diffusion apparatus in accordance with Embodiment 4 of the invention and an illustration showing an example of installing the apparatus.
Figs. 6(a) and 6(b) are a schematic views showing a structure of a chemical diffusion apparatus in accordance with Embodiment 5 of the invention and an illustration of a whole structure of a chemical diffusion system.
Fig. 7 is a schematic view showing a structure of a chemical diffusion apparatus in accordance with Embodiment 6 of the invention.
Fig. 8 is a flowchart of an initially setting operation of the chemical diffusion apparatus in Fig. 7.
Fig. 9 is a schematic view of a structure of a chemical diffusion apparatus in accordance with Embodiment 7 of the invention.
Fig. 10 illustrates a flow of collection and recycle of a chemical cartridge in Fig. 7.
Fig. 11 is a schematic view of a structure of a chemical diffusion apparatus in accordance with Embodiment 8 of the invention.
Figs. 12(a) - 12(c) illustrate a chemical cartridge in Fig. 11.
Fig. 13 illustrates a chemical diffusion unit in Fig. 11.
Figs. 14 (a) and 14 (b) illustrate an operation of mounting a chemical cartridge of a chemical diffusion apparatus in Fig. 11.
Figs. 15(a) and 15(b) illustrate another example of a chemical supply tube in Fig. 11.
Figs. 16 (a) and 16 (b) are a schematic views of a structure of a chemical diffusion apparatus in accordance with Embodiment 9 of the invention.
Figs. 17(a) - 17(c) are a conceptual views showing respective modifications of the chemical diffusion apparatuses shown in Figs. 11 to 16.
Figs. 18(a) and 18(b) are a conceptual views showing an example of a means for discharge a chemical in the chemical diffusion apparatuses shown in Figs. 11 and 16.
Figs. 19(a) - 19(c) are a conceptual views showing respective modifications of the chemical diffusion apparatus in Fig. 16.

### Detailed Description of the Preferred Embodiments

Respective embodiments to which the invention is applied are descried hereinafter, made reference to the drawings.

### (Embodiment 1)

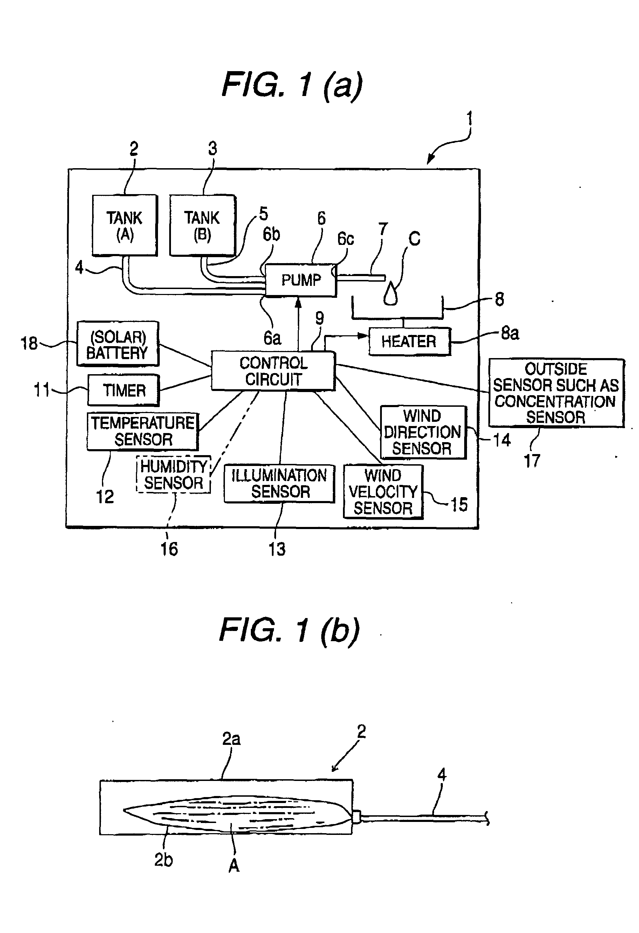

Fig. 1(a) is a schematic view showing a structure of a chemical diffusion apparatus suitable for diffusing a chemical such as a communication disturbance agent and an attractant in a greenhouse or the like. A chemical diffusion apparatus 1 comprises a first tank 2 filled up with a volatile chemical A and a second tank 3 filled up with a volatile chemical B. The tanks 2 and 3 are filled with the chemicals A and B in a solid or liquid state. The chemicals A and B are supplied from the tanks 2 and 3 to suction holes 6a and 6b of a discharge volume variable type pump 6 via chemical supply paths 4 and 5. Each chemical sucked to the pump 6 is mixed in the pump 6, and then, discharged from a discharge tube 7 connected to a discharge hole 6c of the pump 6. A mixed chemical C is discharged from the discharge tube 7 onto an evaporating dish 8 used as a diffusing means. The mixed chemical C discharged onto the evaporating dish 8 is naturally diffused. A heater 8a may be mounted to the evaporating dish 8 for heating within a range that the insect sex pheromone is not changed in characteristic to control a rate of diffusion. Further, the diffusionmaybe controlled by using ultrasonic vibration for spraying instead of a heater since the insect sex pheromone is, in general, easily affected by heat as described above.

A control circuit 9 performs driving control of the pump 6. An input side of the control circuit 9 is connected to a timer 11 (a time detecting means) and a temperature sensor 12, an illumination sensor 13, a wind direction sensor 14 and a wind velocity sensor 15 as environment parameter detecting means. A humidity sensor 16 may be connected. Moreover, an outside sensor 17 such as a concentration sensor for detecting the concentration of a chemical is also connected as the environment parameter detecting means. The control circuit 9 includes a microcomputer and the like and controls drive of the pump 6 on the basis of detecting results from the respective sensors in accordance with a control program stored in advance. That is to say, the control circuit 9 controls discharge of the mixed chemical C from the pump 6 and discharge volume thereof. The control circuit 9 also controls drive of the heater 8a. It is possible to use an outside power source as the power supply for driving each part. As shown in Fig. 1(a), however, using a battery power source 18 such as a solar battery has an advantage that there is no limitation in place for installation.

Fig. 1(b) is a sectional view showing a structure of the first tank 2. The first tank 2 in the embodiment comprises a hard case 2a having an ultraviolet-rays cutoff characteristic and made from a plastic material and a flexible chemical bag 2b built in the hard case 2a. The chemical bag 2b is formed from a flexible material having an oxygen non-transmission characteristic, the flexible material being formed by piling a vacuum-evaporated aluminum film on a plastic film, for example. The second tank 3 is formed similarly. Further, chemical tubes forming the chemical supply paths 4 and 5 are formed from a material capable of cutting off an ultraviolet rays and oxygen. A case of the pump 6 is also formed from a similar material.

In the chemical diffusion apparatus 1 having such a structure, used is a communication disturbance agent for an insect sex pheromone as the chemicals A and B, and the chemical diffusion apparatuses 1 are installed in a farm or the like with every predetermined space. The communication disturbance agent is required to be diffused in accordance with the communication time of male and female noxious insects subject to extermination. In Embodiment 1, the control circuit 9 drives the pump 6 to discharge the chemical C on the basis of the time counted by means of a timer 11. Accordingly, the pump 6 can be driven only in a time period or zone necessary to discharge the chemical, so that the chemical can be diffused effectively, and thereby, mating of the noxious insects can be effectively held down. In other words, unnecessary chemical diffusion can be reduced, and therefore, the maximum effect can be achieved with the minimum amount of chemical.

The control circuit 9 controls drive of the pump 6 in accordance with a temperature, a wind direction, a wind velocity, illumination and such. For example, the discharge volume is controlled to be increased when the wind velocity is high. Controlling the discharge volume as described above allows the concentration of the chemical, which is to be diffused, to be optimum for disturbing communication.

Further, the tanks 2 and 3 are filled with the chemicals A and B under a sealed condition. Accordingly, the chemicals do not decrease due to natural diffusion differently from the case of using a conventional ventilation type tank. In the case that the respective tanks 2 and 3 are of a cartridge type, a filling-up operation becomes easier when the chemical empties. In addition, changing the empty tank with a tank filled up with another chemical can be done immediately when the subject kind of noxious insect is changed.

The tanks 2 and 3, the chemical supply paths 4 and 5 and the case of the pump 6 are formed from a material having an ultraviolet-rays cutoff characteristic and an oxygen non-transmission characteristic. Accordingly, neither antioxidant nor ultraviolet-rays stabilizer is required even in the case that the chemicals A and B are agents that are easily oxidized and deteriorated by ultraviolet rays, that is, a communication disturbance agent for an expensive insect sex pheromone or the like such as aldehyde, for example.

In Embodiment 1, a discharge volume variable type pump is used as a means for discharging a chemical. It may be possible to use a chemical discharge mechanism similar to an ink jet head used in an ink jet printer or the like instead of the above. For example, the chemical discharge mechanism may comprise a chemical pressure chamber supplied with a chemical from a chemical tank, a chemical discharge nozzle communicating with the chemical pressure chamber and an actuator for generating pressure variation of the chemical in the chemical pressure chamber to discharge liquid drops of the chemical from the chemical discharge nozzle. For the actuator, used can be an electrostatic actuator using electrostatic power generated between opposing electrodes, a piezoelectric device or the like. The chemical discharge mechanism having such a structure can be also used in the following chemical diffusion apparatuses.

### (Embodiment 2)

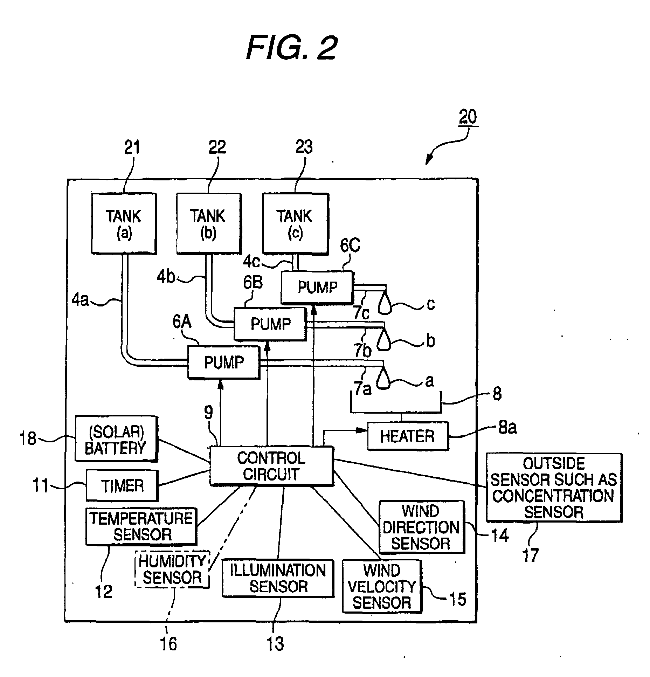

Fig. 2 is a schematic view showing a structure of a chemical diffusion apparatus in accordance with Embodiment 2. A chemical diffusion apparatus 20 comprises a first tank 21, a second tank 22 and a third tank 23, which are used as a chemical tank and which are respectively filled up with chemicals a, b and c different in constituent. Three pumps 6A to 6C are provided for sucking the respective chemicals a to c through chemical supply paths 4a to 4c, respectively, and discharging the same from discharge tube 7a to 7c. The chemicals a to c are discharged onto a common evaporating dish 8 from the respective discharge tubes 7a to 7c. The control circuit 9 is arranged to be able to control drive of the respective pumps 6A to 6C independently. A structure other than the above is omitted from description since it is similar to that of the chemical diffusion apparatus 1 shown in Fig. 1.

In the chemical diffusion apparatus 20 having the above structure, the respective tanks 21 to 23 are filled with communication disturbance agents for an insect sex pheromone or the like, which respectively include different effective constituents. Using the control circuit 9 to selectively drive the pumps 6A to 6C allows a necessary communication disturbance agent to be discharged for diffusion. Furthermore, driving two or more of the pumps 6A to 6c simultaneously as well as adjusting the discharge volume thereof allows communication disturbance agents having different rates of compounded constituents to be diffused. Accordingly, it is possible to diffuse communication disturbance agents effectively against respective noxious insects of different kinds. Increasing the number of the tanks also enables communication disturbance agents to be diffused so as to be effective against noxious insects of much more kinds, of course.

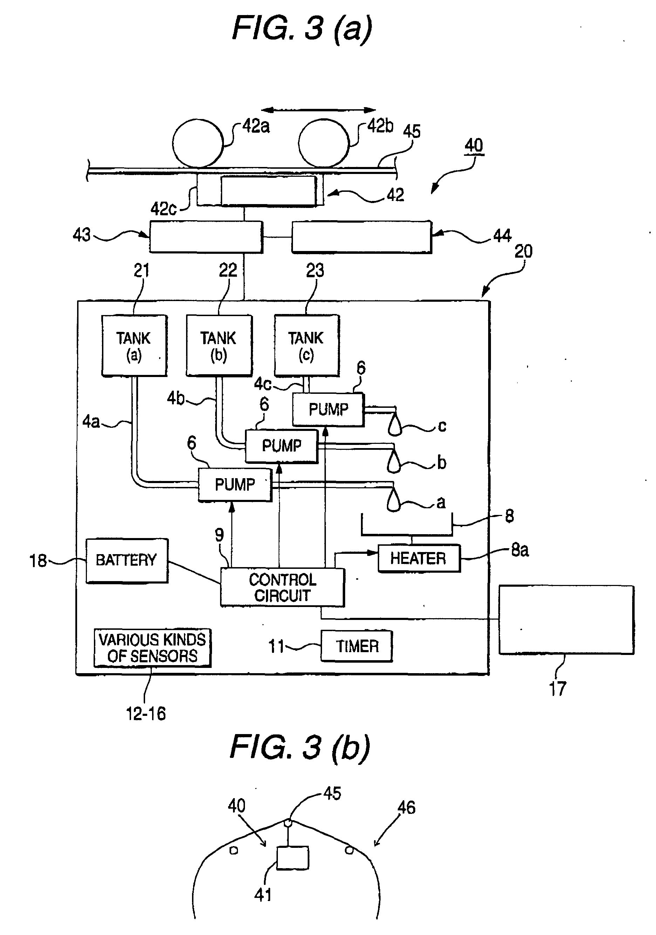

Fig. 3(a) is a schematic view showing a structure of a chemical diffusion system in which the chemical diffusion apparatus 20 having the above structure is used. A chemical diffusion system 40 comprises the chemical diffusion apparatus 20, a travel mechanism 42 to which the chemical diffusion apparatus 20 is mounted, a travel control circuit 43 and a travel position sensor 44. The travel mechanism 42 comprises a pair of front and back travel wheels 42a and 42b, which travel on a travel rail 45, and a pedestal 42c hung from the travel wheels 42a and 42b. The chemical diffusion apparatus 20 is mounted with being hung on the pedestal 42c.

As shown in Fig. 3(b), a travel rail 45 is provided in a longitudinal direction on a ceiling surface of a greenhouse 46. The travel control circuit 43 controls the travel mechanism 42 on the basis of a signal from the travel position sensor 44 comprising an encoder and the like. This causes reciprocative movement of the chemical diffusion system 40 at a predetermined speed in the greenhouse 46 along the travel rail 45.

Using the travel type chemical diffusion system 40 having such a structure allows a chemical to be diffused for the whole area subject to diffusion such as a greenhouse at an even concentration. Accordingly, it is not necessary to install a large number of chemical diffusion apparatuses.

The chemical diffusion apparatus 20 may be hung on a balloon or such to float in a closed space such as a greenhouse. In this case, using a blower or the like to regularly change a wind direction in the greenhouse, for example, allows the chemical diffusion apparatus 20 to be moved.

It is also possible to use the chemical diffusion apparatus 1 in Fig. 1 or later-mentioned respective chemical diffusion apparatuses instead of the chemical diffusion apparatus 20.

### (Embodiment 3)

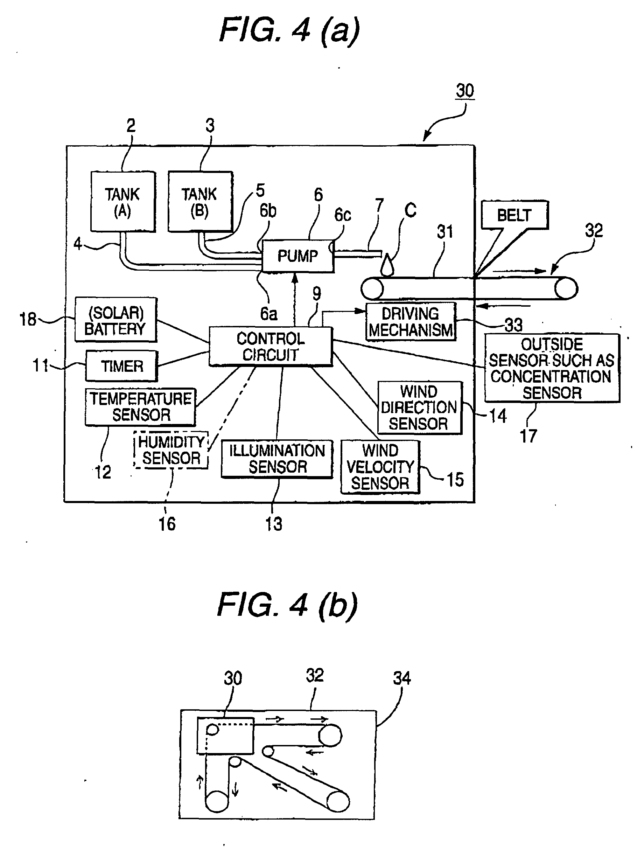

Fig. 4(a) is a schematic view showing a structure of a chemical diffusion apparatus in accordance with Embodiment 3. A basic structure of a chemical diffusion apparatus 30 in Embodiment 3 is similar to that of the chemical diffusion apparatus 1 shown in Fig. 1. Accordingly, same reference numerals and signs are given to the corresponding parts and description of the parts is omitted.

The chemical diffusion apparatus 30 comprises a belt conveyor type diffusing means instead of an evaporating dish. That is to say, the chemical diffusion apparatus 30 comprises a belt conveyor 32 over which an endless belt 31 having a surface material capable of absorbing and carrying chemicals such as felt or sponge, for example, are provided and a driving mechanism 33 for driving the belt conveyor 32. The mixed chemical C is discharged from the discharge tube 7 onto the endless belt 31. When the belt conveyor 32 is driven, the endless belt 31 carrying the mixed chemical C is moved along a predetermined transportation path to diffuse the mixed chemical C along the transportation path of the endless belt 31.

Accordingly, installing the chemical diffusion apparatus 30 at a corner of a greenhouse or a farm ground 34 to lay the belt conveyor 32 in every direction as shown in Fig. 4(b), for example, allows the chemical to be diffused all over the ground 33.

It is also possible, of course, to use the belt conveyor 32 instead of the evaporating dish 8 in the chemical diffusion apparatus 20 shown in Fig. 2.

### (Embodiment 4)

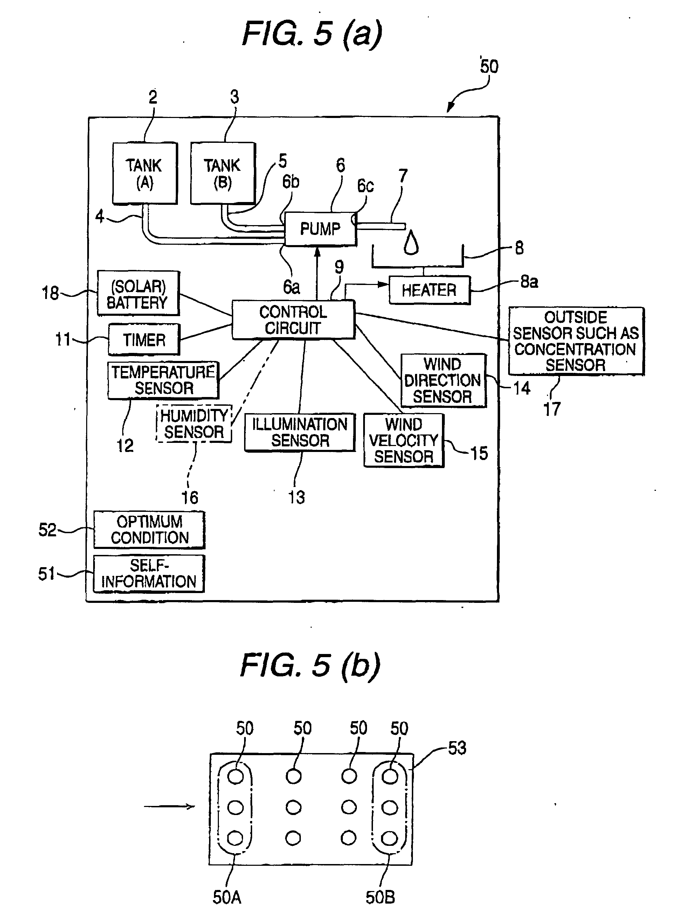

Fig. 5(a) is a schematic view showing a structure of a chemical diffusion apparatus in accordance with Embodiment 4 of the invention. A basic structure of the above is similar to that of the chemical diffusion apparatus 1 shown in Fig. 1. Accordingly, same reference numerals and signs are given to the corresponding parts and description of the parts is omitted. A different point is that the chemical diffusion apparatus in accordance with Embodiment 4 comprises a self-information memory part 51 and an optimum condition memory part 52. The control circuit 9 controls drive of the pump 6 on the basis of information held in the memory parts 51 and 52. The self-information memory part 51 memorizes and holds information relating to a place of installing a chemical diffusion apparatus 50, for example.

The chemical diffusion apparatuses 50 having such a structure are installed in a grid shape with predetermined spaces in a farm ground 53 or the like subject to chemical diffusion, for example, as shown in Fig. 5(b). In this case, when the respective chemical diffusion apparatuses 50 are the same in chemical discharge volume, the chemical concentration at respective places is different in accordance with a wind direction, so that the chemical cannot be diffused evenly. For example, when the wind direction is a direction shown by an arrow in the drawing, the chemical discharged for diffusion from a chemical diffusion apparatus group 50A on the upwind side flows toward a chemical diffusion apparatus group 50B on the downwind side. Accordingly, the chemical diffusion concentration becomes higher at a place on the downwind side.

The self-information memory part 51 of each chemical diffusion apparatus 50 memorizes and holds information relating to a place of installation while the optimum condition memory part 52 memorizes and holds the optimum condition for the chemical discharge volume obtained in accordance with a combination between a wind direction and a place of installation. Accordingly, the control circuit 9 drives the pump 6 so as to satisfy the optimum condition of the chemical discharge volume obtained from the optimum condition memory part 52 on the basis of a wind direction detected by means of the wind direction sensor 14 and a place of installation obtained from the self-information memory part 51, and thereby, discharges the optimum amount of chemical.

When the other conditions are same, the chemical discharge volume from the chemical diffusion apparatus group 50A on the upwind side is more than the chemical discharge volume from the chemical diffusion apparatus group 50B on the downwind side in the case that the wind direction is as shown in Fig. 5(b). As a result, the chemical can be diffused at an almost even concentration all over the ground 53.

### (Embodiment 5)

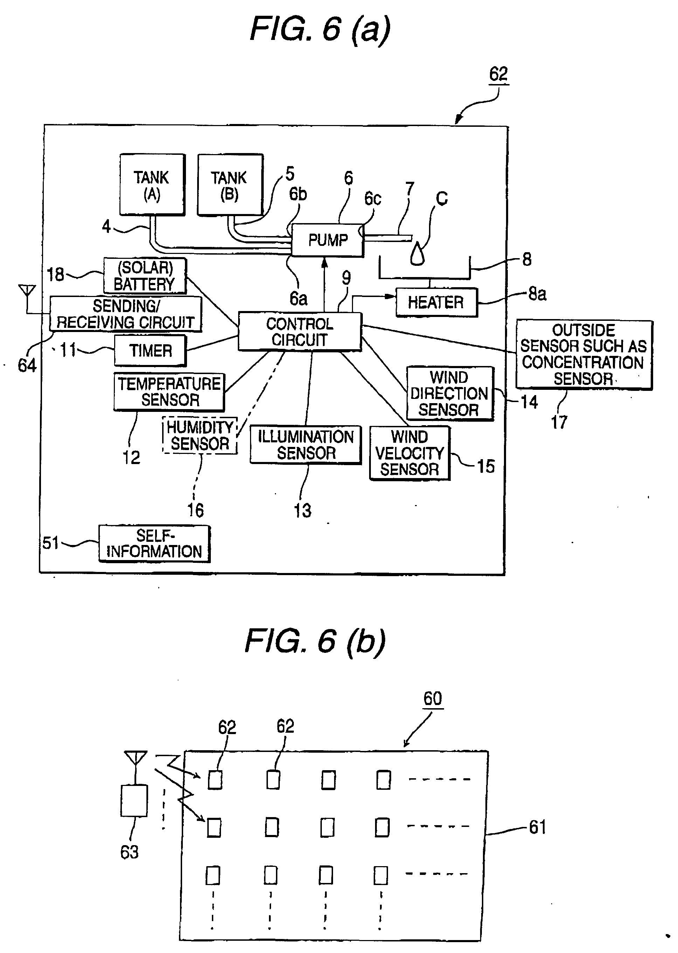

Fig. 6(a) is a schematic view showing a structure of a chemical diffusion apparatus 62 in accordance with Embodiment 5 of the invention. A basic structure of the chemical diffusion apparatus 62 is similar to that of the chemical diffusion apparatus 1 shown in Fig. 1. Accordingly, same reference numerals and signs are given to the corresponding parts and description of the parts is omitted. A different point is that the chemical diffusion apparatus 62 comprises a self-information memory part 51 for memorizing and holding a place of installation and an address (an apparatus ID) and a sending/receiving circuit 64.

Fig. 6(b) illustrates a chemical diffusion system using the chemical diffusion apparatus 62. A chemical diffusion system 60 comprises plural chemical diffusion apparatuses 62 installed with predetermined spaces in a ground 61 subject to chemical diffusion and a radio control apparatus 63 for remotely radio-controlling the chemical diffusion apparatuses 62. The radio control apparatus 63 calculates the optimum driving condition for every chemical diffusion apparatus on the basis of an environment parameter such as a wind direction and a temperature, which is provided by the respective chemical diffusion apparatuses 62, and information relating to a place of installation and an address, and sends the calculated condition to the respective chemical diffusion apparatuses 62. In the respective chemical diffusion apparatuses 62, the control circuit 9 drives the pump 6 on the basis of a received signal to discharge a chemical. Such a structure can also contribute to diffusion of a chemical at an even concentration all over the ground 61 without any influence of a wind direction or the like, similarly to the case of the chemical diffusion apparatus 50 shown in Fig. 5.

### (Embodiment 6)

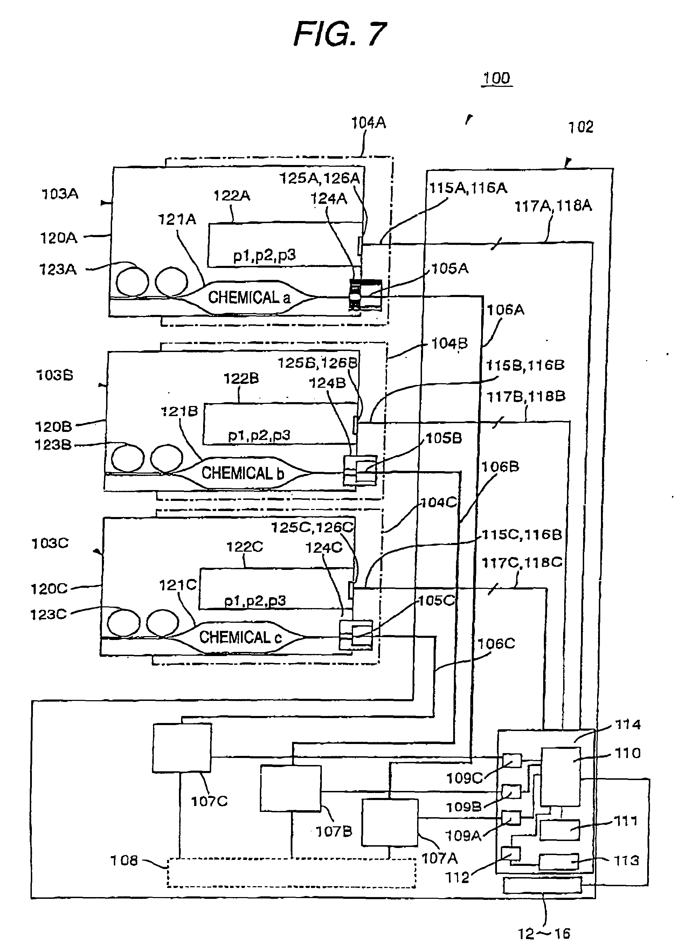

Fig. 7 is a schematic view showing a structure of a chemical diffusion apparatus in accordance with Embodiment 6 of the invention. A chemical diffusion apparatus 100 is provided in respective places in field facilities for extermination of noxious insects. The chemical diffusion apparatus 100 comprises a chemical diffusion unit 102 and a plurality of, three in Embodiment 6, chemical cartridges 103A, 103B and 103C. The chemical diffusing unit 102 comprises three cartridge mounting parts 104A, 104B and 104C for changeably mounting the chemical cartridges 103A to 103C respectively. The respective cartridge mounting parts 104A to 104C are provided with chemical supply holes 105A to 105C, respectively. The chemical supply holes 105A to 105C communicate with sucking ports of chemical discharge pumps 107A to 107C through chemical supply tubes 106A to 106C. Chemicals discharged from the discharge ports of the respective chemical discharge pumps 107A to 107C are supplied to a diffusing dish 108 for naturally diffusing the chemicals in the air. The chemical diffusion unit 102 further comprises pump drivers 109A to 109C for driving the respective chemical discharge pumps 107A to 107C, a control part 110 comprising a microcomputer for controlling the pump drivers 109A to 109C and a unit side memory part 111. The chemical diffusion unit 102 also comprises a timer 112 for determining timing of chemical discharge from the chemical discharge pumps 107A to 107C and a battery 113 for driving the timer. The parts 109A to 109C, 110, 111, 112 and 113 are mounted to a circuit substrate 114.

The respective cartridge mounting parts 103A to 103C are provided with feeding connection terminals 115A to 115C and information reading connection terminals 116A to 116C in addition to the chemical supply holes 105A to 105C. The connection terminals 115A to 115C and the connection terminals 116A to 116C are connected to the circuit substrate 114 via electric power lines 117A to 117C and signal lines 118A to 118C.

The respective chemical cartridges 103A to 103C have a same basic structure. In the inside of cartridge cases 120A to 120C, built in are chemical reservoir parts 121A to 121C in which the chemicals a to c are reserved, cartridge side memory parts 122A to 122C memorizing and holding the optimum discharge conditions for the chemicals a to c or the like and battery power sources 123A to 123C. At the front of the cartridge cases 120A to 120C, provided are chemical supply holes 124A to 124C, feeding connection terminals 125A to 125C and information reading connection terminals 126A to 126C. When the respective chemical cartridges 103A to 103C are mounted to any of the cartridge mounting parts 104 to 104C, the chemical supply holes 124A to 124C of the respective chemical cartridges 103A to 103C, the connection terminals 125A to 125C and 126A to 126C are respectively connected to the chemical supply holes 105A to 105C of the cartridge mounting parts, the connection terminals 115A to 115C and connection terminals 116A to 116C.

In Embodiment 6, the respective cartridge side memory parts 122A to 122C memorize and hold a control parameter for driving a chemical discharging operation, the parameter including a kind p1, a remaining amount p2 and the optimum discharge condition p3 of the chemicals a to c. For memorizing and holding the parameter, a ROM, a RAM, a bar code, an RFID (a radio tag), magnetism and a mechanical shape (protrusion, convex and concave and the like) can be used. In Embodiment 6, a ROM and a RAM are used while a connection is a cable connection.

In Embodiment 6, the chemicals a, b and c are an insect sex pheromone and the optimum discharge condition p3 includes the discharge starting time, a discharge interval, the discharge volume, the discharge ending time and the like of the chemical discharge pumps 107A to 107C. Satisfying such discharge conditions allows the insect sex pheromone to be diffused at a proper diffusion concentration in the vicinity of a place of installing the chemical diffusing unit 102 at the proper time. The chemical diffusion unit 102 comprises at least one of the environment parameter detecting means (12 to 17) similar to the respective chemical diffusion apparatuses shown in Figs. 1 to 6. The control part 110 controls drive of the chemical discharge pumps 107A to 107C on the basis of the detection result. Accordingly, the chemical can be diffused under the optimum condition corresponding to an environment of the place of installation, similar to the case of the chemical discharge pumps in Figs. 1 to 6.

For the respective chemicals a, b and c, used can be a communication disturbance agent sold commercially, which is described in Page 72 of "PHEROMONE AGENT UTILIZATION GUIDE" (issued by JAPAN PLANT PROTECTION ASSOCIATION (SHADANHOUJIN NIHON SHOKUBUTSU BOEKI KYOKAI), for example. As an example, a comparison table between constituents of CONFUSER A and CONFUSER P and a subject noxious insect is shown as follows.

| Name of Chemical | CONFUSER A | CONFUSER P | Subject Noxious Insect |
|---|---|---|---|
| *Arimarua* | 42 % | - | *Phyllonorycter ringoniella* |
| *Orifurua* | 4.5 % | 20 % | *Grapholita molesta* |
| *Tetradecenylacetate* | 21 % | 16 % | *Leaf Rollers* |
| *Peachfurua* | 9.5 % | 17 % | *Carposina sasakii Matsumura* |
| *Pirimarua* | - | 25 % | *Lyonetia clerkella Linne* |
| Stabilizer | 23 % | 22 % | |
| Consumed Amount per 10 a | 200 units | 180 units | |

The chemicals a, b and c can be a compound of various kinds of chemicals like CONFUSER A, but it may be arranged that the chemical a be *Arimarua*, the chemical b be *Tetradecenylacetate*, and the chemical c be *Peachfurua,* for example.

In the chemical diffusion apparatus 100 having such a structure, when the chemical cartridges 103A to 103C are respectively inserted into the cartridge mounting parts 104A to 104C, they are connected to corresponding parts of the chemical diffusion unit 102 in order from the chemical supply holes 124A to 124C, information reading connection terminals 126A to 126C and feeding connection terminals 125A to 125C. The respective cartridge mounting parts 104A to 104C are provided with a cartridge sensor (not shown) for detecting that the chemical cartridges 103A to 103C are properly mounted. The control part 110 can determine whether the chemical cartridges 103A to 103C are mounted or not on the basis of an output from each cartridge sensor.

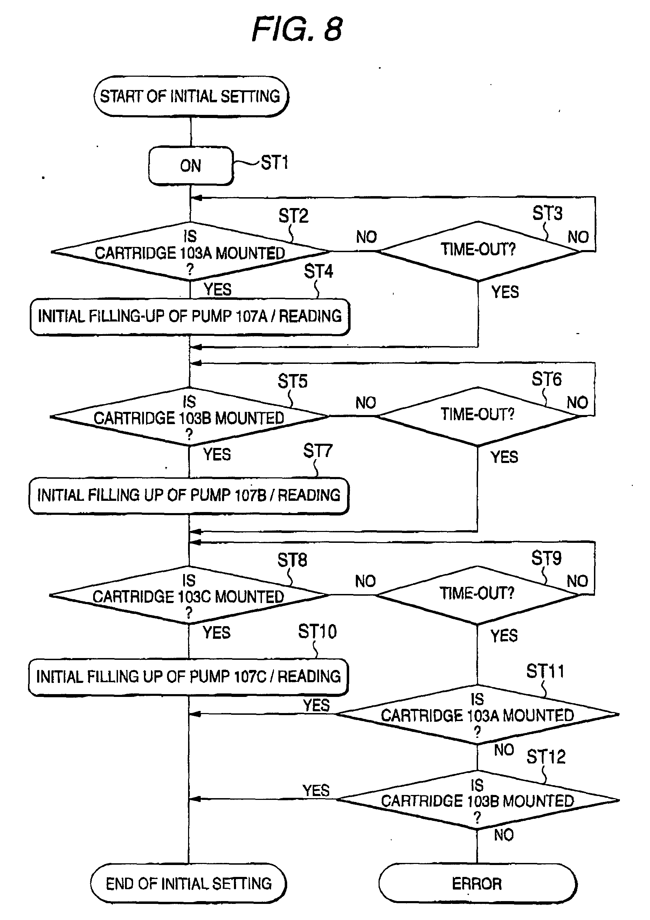

Fig. 8 is a schematic flowchart showing an initial setting operation when the chemical cartridge is mounted. In Embodiment 6, when the chemical cartridge 103A is inserted to the cartridge mounting part 104A, it is connected to the chemical supply holes 124A, the information reading connection terminal 126A and the feed connection terminal 125A in this order, as described above. When the feed connection terminal 125A is connected, the chemical diffusion apparatus 100 is turned on (Step ST1). After the chemical cartridge 103A is detected to be properly mounted (Step ST2), the chemical diffusion apparatus 100 drives the chemical discharge pump 107A to carry out initial filling-up of the chemical a as well as reading-out of the information stored in the cartridge side memory part 122A of the chemical cartridge 103A to store the read-out information in the unit side memory part 111 (Step ST4). Similarly, in the case of inserting the chemical cartridges 103B and 103C, an operation for detecting whether or not the cartridges are properly mounted is also carried out (Steps ST5 and ST6 and Steps ST8 and ST9) to perform initial filling-up and reading-out of information as well as the above case (Steps ST7 and ST10).

In the case that the chemical cartridge 103C is not mounted within a predetermined time period, an initial setting operation is completed when any one of the chemical cartridges 103A and 103B is mounted (Steps ST11 and ST12). When all of the chemical cartridges 103A, 103B and 103C are not mounted within a predetermined time period, an operation for displaying an error of the cartridge, for example, is carried out since no initial setting can be performed.

The chemical diffusion apparatus 100 enters a sleep mode after the initial filling-up. Then, the chemical diffusion apparatus 100 refers to the optimum discharge conditions read out from the respective chemical cartridges 103A to 103C to wake up at the discharge starting time and check existence, a kind and remaining volume of the cartridge, and then, discharges the respective chemicals a to c at the optimum discharge interval and discharge volume until the ending time. In the above context, the optimum discharge conditions are for achieving chemical diffusion at the optimum concentration by starting discharge from just before sunset to maintain the same until the predetermined ending time with a discharge interval and a discharge volume, which are optimum for disturbing communication of subject noxious insects, since a mating behavior of insects is generally active after sunset until night. The sunset time varies in accordance with a place and a season. Therefore, calendar information for the optimum discharge, such as a condition in May, a condition in August ..., is preferably stored to accommodate variations in accordance with a season. The chemical diffusion apparatus 100 may be provided with a sensor such as an illuminometer as an environment parameter detecting means to detect the sunset time on the basis of the illumination, and thereby, start chemical diffusion from the sunset.

The control part 110 of the chemical diffusion unit 102 in Embodiment 6 calculates a chemical consuming amount on the basis of the number, time or the like of discharge from the chemical discharge pump to subtract the calculated value from a remaining amount p2 read out from the respective chemical cartridges, and thereby, calculate a chemical remaining amount in the respective chemical cartridges 103A to 103C. The calculated chemical remaining amount is written in the cartridge side memory parts 122A to 122C of the respective chemical cartridges 103A to 103C with proper frequency to renew the chemical remaining amount. This results in the optimum discharge operation without any trouble even in the case that the chemical cartridges 103A to 103C are temporally detached from the cartridge mounting parts 104A to 104C to be mounted again or mounted to another chemical diffusion apparatus.

As described above, in the chemical diffusion apparatus 100, simply mounting the chemical cartridges 103A to 103C in which desired chemicals are reserved to the cartridge mounting parts 104A to 104C of the chemical diffusion unit 102 allows the chemical diffusion unit 102 to carry out a discharge operation optimum for the chemicals reserved in the chemical cartridges 103A to 103C. Further, even in the case that a new chemical is developed, giving the latest information to the chemical cartridges 103A to 103C allows no change in drive controlling program of the chemical diffusion unit 102 to be required.

In the chemical diffusion apparatus 100, it is convenient to enable an individual condition (location information) to be set in the unit side memory part 111 of the chemical diffusion unit 102. For example, information relating to the installation location of the chemical diffusion unit 102 is stored and utilized as described in the following situations (1) and (2) while the chemical diffusion unit 102 performs chemical discharge control described in parentheses. This enables unnecessary chemical diffusion to be prevented and allows a chemical to be diffused in a location at an even concentration.
(1) On the upper part (setting the discharge volume more) or the lower part (setting the discharge volume less) of a slant surface
(2) In a relative position in the field (setting the discharge volume more on the upwind side and setting the discharge volume less on the downwind side)

### (Embodiment 7)

In the chemical diffusion apparatus 100 in Embodiment 6, it is necessary to change and reset a battery since a battery 113 for a timer is mounted to the chemical diffusion unit 102. In the case of a place like Northern Japan where there is a season such as a winter when neither crops nor noxious insects exist, it is uneconomical to keep the chemical diffusion unit 102 at a driving condition only for the purpose of using the timer during the period. Further, the chemical diffusion apparatus is generally installed in a high place several meters above the ground since the insect sex pheromone is heavier than air. Accordingly, an operation on the spot such as a change of a battery and resetting after a change of a battery is difficult in general.

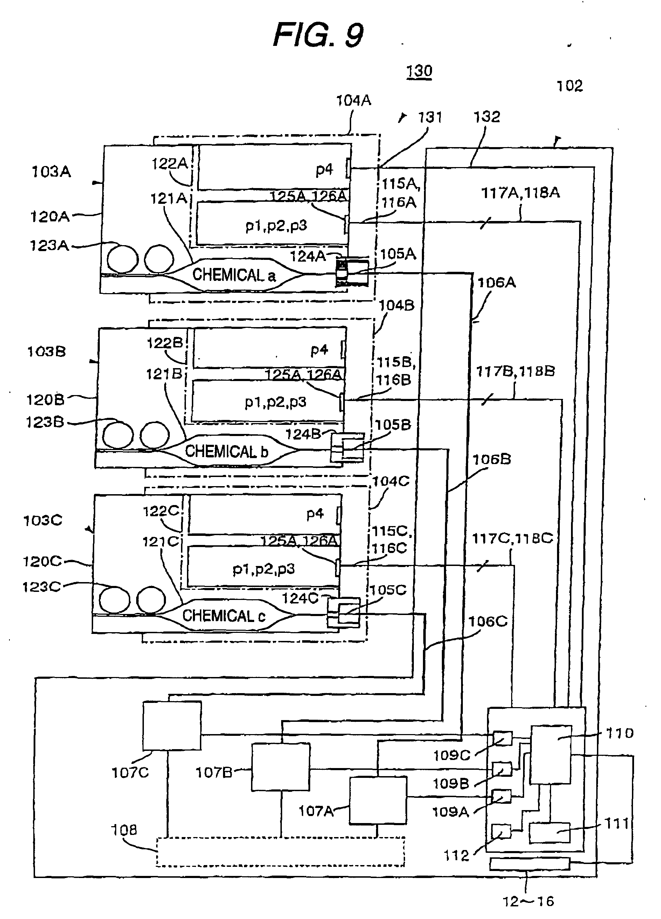

Fig. 9 is a schematic view of a structure of a chemical diffusion apparatus in accordance with Embodiment 7 of the invention. The chemical diffusion apparatus is characterized in that a change of a battery and resetting after a change of a battery are unnecessary and that a drive controlling program of the chemical diffusion apparatus is mounted to the chemical cartridge.

As seen from Fig. 9, a basic structure of a chemical diffusion apparatus 130 is similar to that of the chemical diffusion apparatus 100 in Fig. 7. Accordingly, same reference numerals and signs are given to the corresponding parts and description of the parts is omitted. In the chemical diffusion apparatus 130, a drive controlling program p4 of the chemical diffusion unit 102 is mounted to the cartridge side memory parts 122A to 122C of the respective chemical cartridges 103A to 103C. The drive controlling program p4 includes time information of the timer 112 or the like. It may be possible to store the timer information for individual chemicals in the respective chemical cartridges 103A to 103C to drive the respective chemical discharge pumps 107A to 107C to which the chemicals are supplied from the respective chemical cartridges 103A to 103C by means of independent timers suitable for the respective chemicals in the chemical cartridges 103A to 103C connected to the chemical discharge pumps 107A to 107C. In Embodiment 7, however, the first priority is given to the cartridge mounting part 104A, which is the only cartridge mounting part that is provided with a terminal 131 and a lead wire 132 capable of reading the drive controlling program p4. Selection of a drive controlling program mounted to any one of the chemical cartridge parts 104A to 104C may be determined on the basis of the mounting order instead of the order of priority of the cartridge mounting parts 104A to 104C. For example, the drive controlling program p4 may be read from one of the chemical cartridges 103A to 103C, which is first or last mounted to one of the cartridge mounting parts 104A to 104C. Otherwise, it is possible to put the chemical cartridges 103A to 103C in order of priority to read a drive controlling program mounted to the chemical cartridge with the first priority among the mounted chemical cartridges.

As described above, in the chemical diffusion apparatus 130, the drive controlling program (information for controlling a discharge pump) p4 of the chemical diffusion unit 102 is mounted on the side of the chemical cartridges 103A to 103C. Accordingly, in order to install the chemical diffusion system 130, for example, only writing information in the respective chemical cartridges 103A to 103C or checking information mounted to the respective chemical cartridges 103A to 103C all together and inserting the chemical cartridges 103A to 103C to the chemical diffusion unit 102 on the spot allow all conditions to be automatically set. This makes manual condition setting by an operator for every chemical diffusion unit 102 unnecessary. Further, only inserting the chemical cartridges 103A to 103C is enough even in the case in which an installed chemical diffusion unit 102 and a chemical diffusion unit 102 to be newly installed are intermingled. This has a large effect.

Moreover, driving power of the chemical diffusion unit 102 is supplied from the battery power sources 123A to 123C of the chemical cartridges 103A to 103C. This makes a battery changing operation and a resetting operation after the change on a chemical diffusion unit 102 side unnecessary.

As the location information memorized in the cartridge side memory parts 122A to 122C of the chemical cartridges 103A to 103C, regional information relating to a place of installation may be included because sunset time and the mating behavior of noxious insects at the same certain times differ between Okinawa and Hokkaido. Accordingly, it may be possible to store standard timer and calendar information as well as time difference adjustment information for adjusting a difference in sunset time among respective regions to discharge chemical liquids under the optimum discharge condition on the basis of the memorized information. It goes without saying that giving the regional information to the unit side memory part 111 of the chemical diffusion unit 102 allows the above-mentioned time difference between Okinawa and Hokkaido to be automatically adjusted by only inputting or selecting a region for installation.

In Embodiments 6 and 7, forming the chemical reservoir parts 121A to 121C of the respective chemical cartridges 103A to 103C from a changeable bag or the like and using a rewritable memory chip such as NVRAM for the cartridge side memory parts 122A to 122C allow recycle of the chemical cartridges 103A to 103C to be easily performed by changing the chemical reservoir parts 121A to 121C and the battery power sources 123A to 123C and rewriting the memory parts 122A to 122C. It is desirable to hold information concerning the customers purchasing the chemical diffusion system in the chemical cartridges in order to give a privilege such as maintenance and/or discount on a purchase price to the customers on the basis of the purchaser information held in the used and collected chemical cartridges in order to increase a rate of collecting the chemical cartridges 103A to 103C for the purpose of recycling.

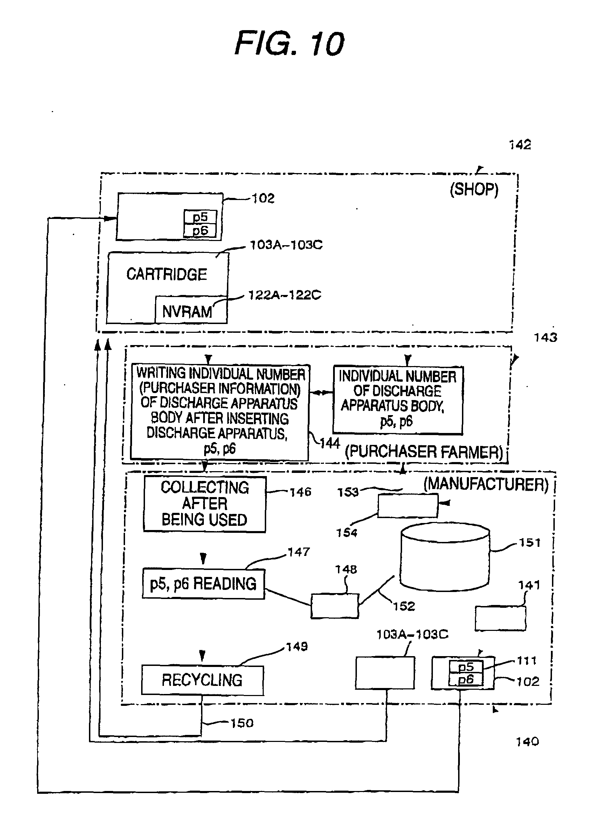

For that purpose, purchaser information p5 is written in the unit side memory part 111 of the chemical diffusion unit 102 by means of a writing apparatus 141 on a chemical diffusion apparatus manufacturer 140 side while a shop 142 sells the chemical diffusion apparatus to the purchaser 143, as shown in Fig. 10. The shop 142 also sells chemical cartridges 103A to 103C of various kinds, which are manufactured by the manufacturer 140, to the purchaser 143. A farmer, which is the purchaser 143, for example, installs the purchased chemical diffusion unit 102 in a predetermined place of installation, purchase and mount the chemical cartridges 103A to 103C containing required chemicals, to the chemical diffusion unit 102. When the chemical cartridges 103A to 103C are mounted, the purchaser information p5 held in the chemical diffusion unit 102 as well as individual information (apparatus identification information) p6 of the chemical diffusion unit 102 is read by the chemical cartridges 103A to 103C to be written in the cartridges side memory parts 122A to 122C of the chemical cartridges 103A to 103C (Block 144).

The farmer 143 collects the used chemical cartridges, and then, returns them to the manufacturer directly or through the shop for recycling. The manufacturer 140 reads the purchaser information memorized in the used and collected chemical cartridges by means of a reading apparatus 148 (Block 147). The manufacturer 140 then recycles the chemical cartridges to sell the same to the shop at wholesale prices (Block 149, Arrow 150).

The manufacturer 140 constructs and renews a purchaser database 151 on the basis of the read purchaser information (Arrow 152). The purchaser database 151 comprises a corresponding table of the apparatus identification information p6 assigned to the chemical diffusion unit 102 and the purchaser information p5. The manufacturer 140 offers various kinds of services to the purchasers 143 on the basis of the constructed purchaser database 151 (Block 154, Arrow 153).

As for contents of the service, considered are a partial refund of a purchase price, discount on a purchase price of the chemical cartridge for the next time, a notice of new products for the next purchase (provided by a DM or via a WEB), provision of the latest agricultural information relating to the used chemical cartridge (or the subject noxious insect), support for advertisement on the WEB managed by the manufacturer such as "ENVIRONMENT-FRIENDLY FIRMS (REDUCTION IN AGRICULTURAL CHEMICAL, CONTRIBUTION TO RECYCLE AND THE LIKE) " and for making a WEB of each farmer, which is the purchaser, database providing services for the purpose of traceability of farm products, etc.

The purchaser information may be written in a chemical cartridge by means of an exclusive writing apparatus during purchase. In addition, it is desirable to encode algorithms of communication between the chemical cartridge and the chemical diffusion unit 102 or a memory structure thereof to prevent the unapproved chemical from being discharged and the purchaser information from being used.

### (Embodiment 8)

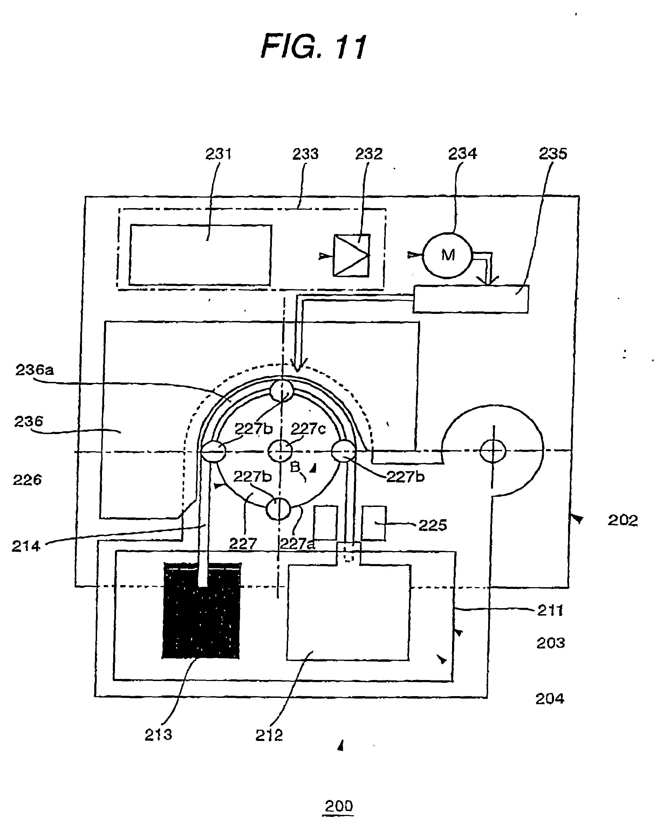

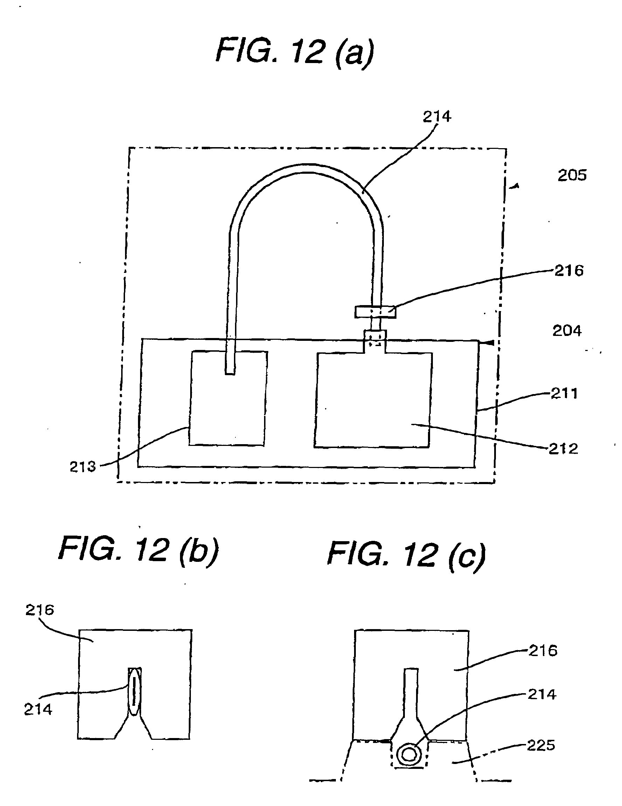

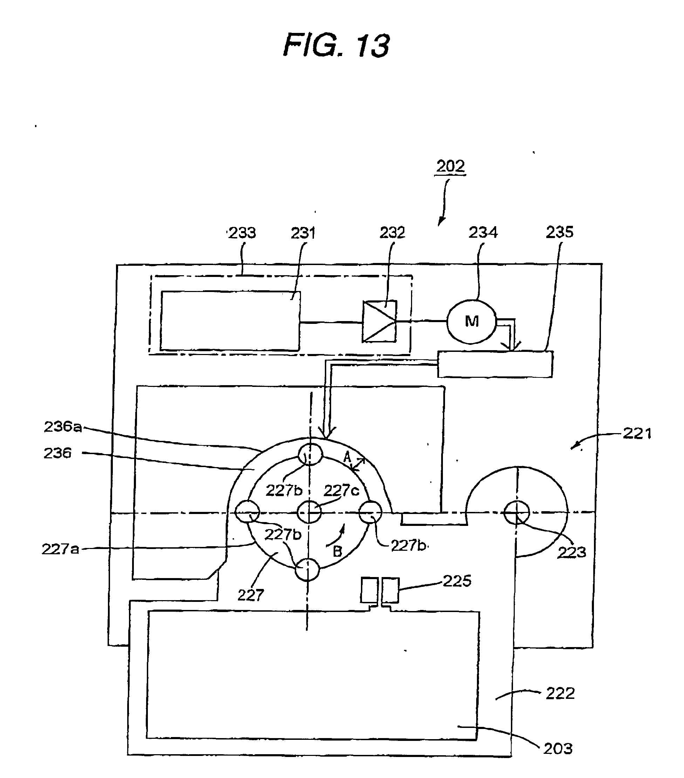

Fig. 11 illustrates a chemical diffusion apparatus for diffusing a chemical such as an insect sex pheromone in accordance with Embodiment 8. Figs. 12(a) -12(c) illustrate a chemical cartridge thereof. Fig. 13 illustrates a chemical diffusion unit of the same. As shown in the drawings, a chemical diffusion apparatus 200 comprises a chemical diffusion unit 202 and a chemical cartridge 204 changeably mounted to a cartridge mounting part 203 formed on the chemical diffusion unit 202.

As shown in Figs. 11 and 12(a), the chemical cartridge 204 comprises a cartridge case 211, a chemical tank 212 and a chemical diffusion part 213, which are mounted to the cartridge case 211, and a chemical supply tube 214 connecting the chemical tank 212 with the chemical diffusion part 213. The chemical diffusion part 213 is formed form a porous material or a fibriform material. An opening for diffusing a chemical (not shown) is formed on a part of the cartridge case 211, the part housing the chemical diffusion part 213. The chemical supply tube 214 is formed from a soft material not expanded by a chemical. A material such as Viton (a product name) made by E. I. DU PONT DE NEMOURS, for example, can be used for forming the chemical supply tube 214.

The chemical cartridge 204 is vacuum-packed with a sealing bag 205 shown by a virtual line in Fig. 12 (a) in shipping. A clip 216 is put on the chemical supply tube 214 drawn from the chemical tank 212. As shown in Fig. 12(b), the clip 216 presses the chemical supply tube 214 to seal the same. When the clip 216 is released, the chemical supply tube 214 returns into a cylindrical shape as shown in Fig. 12(c), and thereby, a chemical can be supplied from the chemical tank 212 to the chemical diffusion part 213.

As shown in Figs. 11 and 13, the chemical diffusion unit 202 comprises a fixed side case 221 and a movable side case 222. The movable side case 222 can rotate in a predetermined range of an angle, 90 degrees, for example, in a horizontal direction about a vertical holding shaft 223 mounted to the fixed side case 221. The movable side case 222 is formed with the cartridge mounting part 203 for changeably mounting the chemical cartridge 204. A protrusion 225 is also formed for pushing up and releasing the clip 216 sealing the chemical supply tube 214 in mounting the chemical cartridge 204. Further, a rotor 227, which is a component of a tube pump 226, is held so as to be freely rotatable.

The fixed side case 221 of the chemical diffusion unit 202 comprises a control unit 233 to which the control circuit 231 and a motor driver 232 are mounted, a motor 234, a power transmission mechanism 235 for transmitting motor rotation power to the tube pump 226 and a stator 236, which is a component of the tube pump 226. The chemical diffusion unit 202 comprises at least one of the environment parameter detecting means (12 to 17), similar to the respective chemical diffusion apparatuses shown in Figs. 1 to 6. The control unit 235 controls drive of the tube pump 226 on the basis of a result of the detection. Accordingly, a chemical can be diffused under the optimum condition corresponding to an environment of a place of installation, similar to the case of the chemical diffusion apparatuses in Figs. 1 to 6.

The tube pump 226 comprises the stator 236 mounted to the fixed side case 221, the rotor 227 mounted to the movable side case 222 and the chemical supply tube 214 mounted to the chemical cartridge 204. The stator 236 is formed with an inner circumferential surface 236a in the shape of an arc. When the movable side case 222 is fixed at a closing position shown in Figs. 11 and 13, a circular outer circumferential surface 227a of the rotor 227 is opposed to the inner circumferential surface 236a of the stator with a fixed gap A over a range of angles of almost 180 degrees. To the gap A, mounted is the chemical supply tube 214 of the chemical cartridge 204. Roller bearings 227b are freely rotatably provided on the outer circumferential surface 227a of the rotor 227 with a fixed space of an angle, the gap of an angle of 90 degrees in the case of the drawings. The roller bearings 227b press the chemical supply tube 214 and simultaneously rotate with the rotor 227 when the rotor 227 rotates in a direction shown by an arrow B about a rotation shaft 227c. The rotation shaft 227c of the rotor 227 is connected to the power transmission mechanism 235 when the movable side case 222 is fixed at the closing position. The rotation shaft 227c is thus rotatably driven by means of a motor 234 through the power transmission mechanism 235. The tube pump 226 having such a structure is well known, and therefore, further description is omitted. Embodiment 8 is characterized in that fixing the movable side case 222 to which the chemical cartridge 204 is mounted at a closing position by means of a locking mechanism not shown allows the tube pump 226 to be formed.

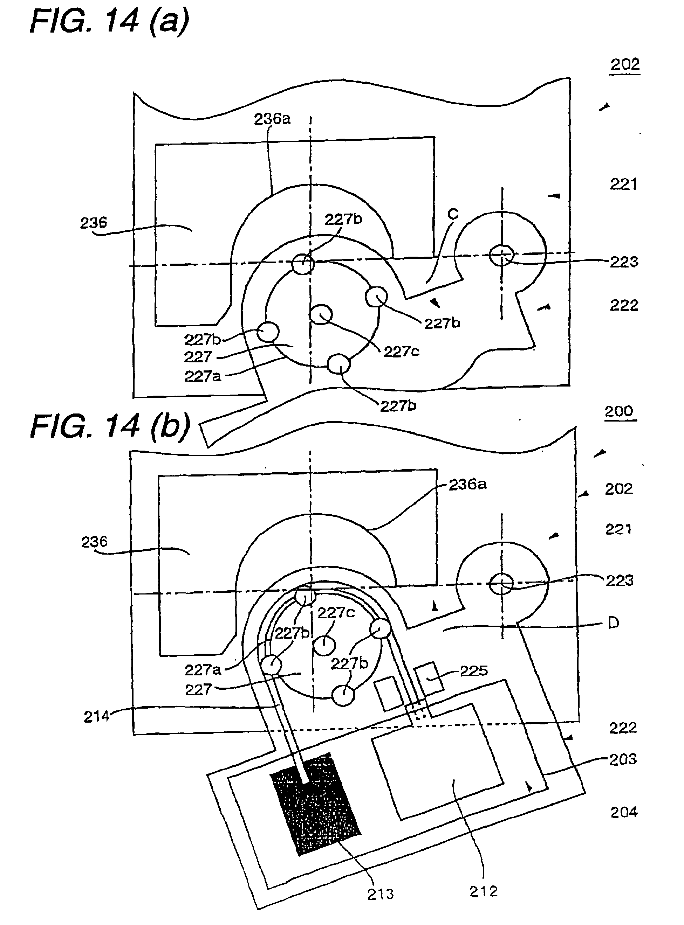

Figs. 14 (a) and 14 (b) illustrate an operation of mounting the chemical cartridge 204 to the chemical diffusion unit 202. In mounting the chemical cartridge 204, the movable side case 222 of the chemical diffusion unit 202 at the closing position (refer to Fig. 13) is opened in a direction shown by an arrow C about the vertical holding shaft 223, as shown in Fig. 14(a). This results in release of the outer circumferential surface 227a of the rotor from the stator inner circumferential surface 236a of the tube pump 226, so that the chemical supply tube 214 can be easily mounted therebetween. Then, the chemical cartridge 204 is mounted to the cartridge mounting part 203 of the open movable side case 222 as shown in Fig. 14(b). As a result, the chemical supply tube 214 is provided along the outer circumferential surface 227a of the rotor 227. In mounting the chemical cartridge 204, the protrusion 225 releases the clip 216 from the chemical supply tube 214, so that the chemical tank 212 is communicated with the chemical diffusion part 213. This enables a chemical to be supplied.

The movable side case 222 is then rotated in a direction shown by an arrow D in Fig. 14 (b) to fix the movable side case 222 at the closing position, as shown in Fig. 11. This results in the chemical cartridge 204 mounted to the chemical diffusion apparatus 2, so that the tube pump 226 is formed. After the chemical cartridge 204 is so mounted, the motor 234 is driven under the control of the control unit 231 in a fixed time period or zone after the sunset during a day, for example. A chemical in the chemical tank 212 is discharged to the chemical diffusion part 213 through the chemical supply tube 214 by means of the tube pump 226 when the motor 234 is driven. The chemical discharged to the chemical diffusion part 213 is naturally diffused in the air. Installing the chemical diffusion apparatus 200 in a predetermined place of installation in a cultivation field such as an orchard enables noxious insects to be exterminated. The chemical discharge volume is counted by means of the control unit 231 on the basis of a rotation number of a rotor of the tube pump 226, for example. Otherwise, it is counted by means of the control unit 231 on the basis of an integrated value of a time period for driving the tube pump.

When the chemical in the chemical tank 212 is emptying, the control unit 231 gives a notice of the fact through an indicator not shown, or the like. An operation of changing the chemical cartridge 204 is performed similarly to the above-mentioned mounting operation. The chemical diffusion part 213 and the chemical supply tube 214 are changed at the same time as the change of the chemical cartridge 204 since they are mounted to the chemical cartridge 204. Accordingly, only changing a chemical cartridge is enough even in the case of diffusing a different kind of chemical, for example, so that no operation of cleaning the chemical diffusion part or the chemical supply tube is required.

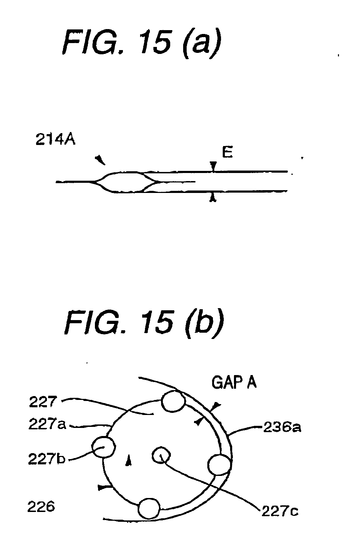

In Embodiment 8, a tube made of metal such as stainless steel may be used for the chemical supply tube 214 of the tube pump 226 instead of a tube made of resin. For example, a flexible metal tube 214A formed by welding both ends of two sheets of stainless steel foil as shown in Fig. 15(a) may be used. A cylindrical metal pipe may be rolled into the shape shown in Fig. 15 (a) instead of the welding of two sheets of metal foil. In this case, arranging the thickness E to be a dimension corresponding to the gap A of the tube pump 226 shown in Fig. 15 (b) allows the volume in a state of compression by the roller bearing 227b and the volume in a state of release from the compression to be stabilized, so that the chemical discharge volume can be stabilized.

The chemical diffusion part 213 and the chemical tank 212, which are mounted to the chemical cartridge 204, may be individually changeable. Only changing the chemical diffusion part 213 is enough when the chemical diffusion part 213 becomes very dirty before the chemical is exhausted. On the other hand, only changing the chemical tank 212 is enough when the chemical becomes empty with the chemical diffusion part 213 being usable. This contributes great economy.

### (Embodiment 9)

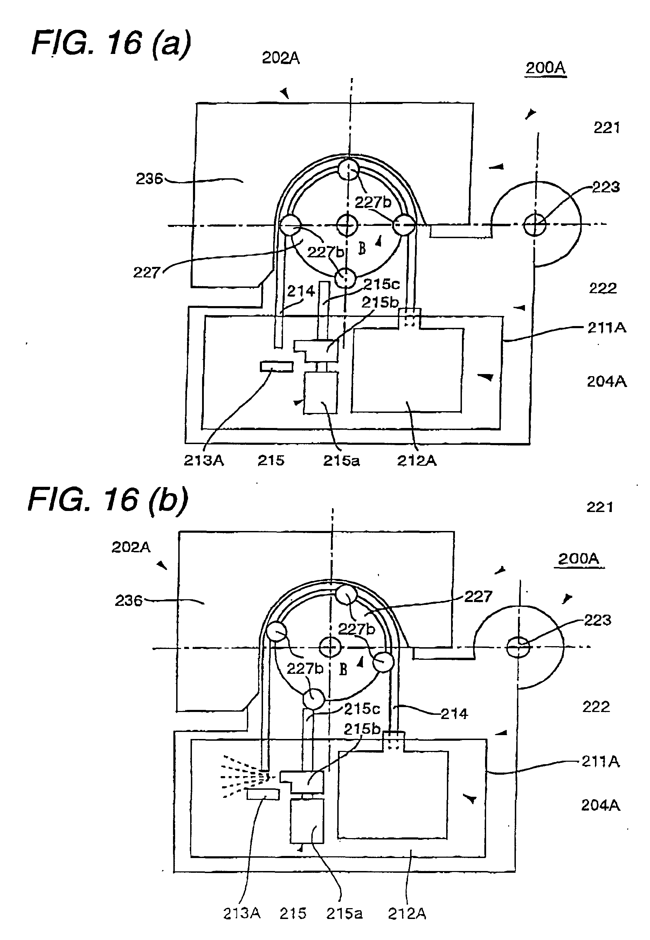

Figs. 16(a) and 16(b) illustrate a chemical diffusion apparatus comprising a chemical cartridge having a structure different from that of the chemical cartridge 204. A chemical diffusion unit of a chemical diffusion apparatus 200A in Figs. 16(a) and 16(b) has a structure same as that of the chemical diffusion unit 202. Accordingly, same reference numerals and signs are given to the corresponding parts and description of the parts is omitted.

The chemical cartridge 204A of the chemical diffusion apparatus 200A comprises a spray type diffusion means as a chemical diffusion means. That is to say, to a cartridge case 211A of the chemical cartridge 204A, mounted are a chemical tank 212A, a receiving dish 213A and a spray can 215 filled up with spray pressured gas for diffusing a chemical discharged from the chemical tank 212A to the receiving dish 213A through the chemical supply tube 214. The spray can 215 comprises a can body 215a, a nozzle 215b mounted on a top end of the can body 215a and an operation rod 215c. The spray can 215 is arranged so that the operation rod 215c would be pressed to open the nozzle 215b, and thereby, the pressurized gas would be blown.

In Embodiment 9, a roller bearing 227b of the tube pump 226 is used for pressing the operation rod 215c. When the rotor 227 rotates in a direction shown by Arrow B, the roller bearings 227b, which project toward the outside, rotate together with the rotor 227. When the rotor 227 rotates in the direction shown by Arrow B, the respective roller bearings 227b intermittently press the operation rod 215c as shown in Fig. 16 (b) since the a top part of the operation rod 215c is located so as to interfere with rotation tracks of the roller bearings 227b. As a result, the nozzle 215b of the spray can 215 intermittently opens to intermittently blow the pressurized gas, so that the chemical discharged onto the receiving dish 213A is diffused in the air. The diffusion time of the pressurized gas can be adjusted in accordance with the rotation speed of the rotor 227.

In accordance with the chemical diffusion apparatus 200A having such a structure, only changing the chemical cartridge 204A allows the chemical to be changed in kind, similarly to the case of the chemical diffusion apparatus 200. Further, it is convenient to changeably mount the spray can 215 to the chemical cartridge 204A since only changing the spray can 215 is enough when the pressurized gas becomes empty. Moreover, it is extremely convenient to also set the chemical tank 212A changeably to the chemical cartridge 204A since both of the chemical and the pressurized gas can be individually changed. This contributes greater economy than the case in which the chemical cartridge 204A should be changed with one of the chemical and the pressurized gas being remained, since the both of the chemical and the pressurized gas can be completely exhausted. In addition, it is not necessary to check affinity whether or not the chemical and the pressurized gas can be mixed for a reserve, differently from the case of filling up a same spray can 215 with the chemical and the pressurized gas, so that the spray type diffusing means can be used for a large number of chemicals. Further, a used spray can 215 is reusable by simply filling it up with the pressurized gas again without cleaning. Accordingly, the spray can 215 used as the pressurized gas reservoir part can be easily reused.

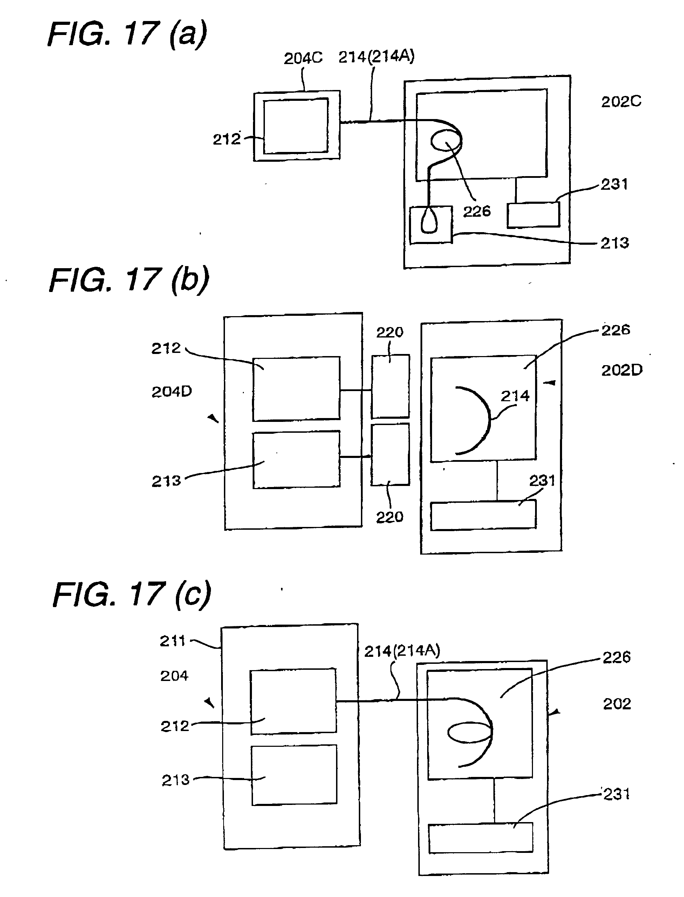

Figs. 17(a) to 19(c) are conceptual views illustrating various modifications of Embodiments 8 and 9. Fig. 17(c) is a conceptual view of the chemical diffusion apparatus 200 in accordance with Embodiment 8. In Fig. 17(c), the chemical diffusion part 213 and the chemical supply tube 214 are mounted to the chemical cartridge 204 together with a chemical tank 212.

It is also possible to only mount the chemical tank 212 and the chemical supply tube 214 to the chemical cartridge 204C and to mount the chemical diffusion part 213 to the chemical diffusion unit 202C, as shown in Fig. 17(a), instead of the above.

Further, the chemical tank 212 and the chemical diffusion part 213 may be mounted to the chemical cartridge 204D with the tube pump 226 being mounted to the chemical diffusion unit 202D, as shown in Fig. 17 (b). In this case, both ends of the chemical supply tube 214 on the chemical diffusion unit 202D side should be connected to the chemical tank 212 and the chemical diffusion part 213 of the chemical cartridge 204D, respectively, through a connecting means 220. The connecting means 220 can be mounted on any side of the chemical diffusion unit 202D and the chemical cartridge 204D.

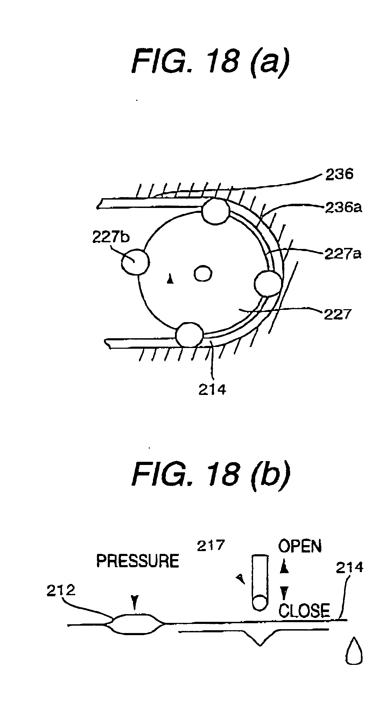

Fig. 18 (a) is a conceptual view showing the tube pump used as a chemical diffusing means in Embodiments 8 and 9. For the chemical diffusing means, a pump other than a tube pump, a cylinder pump or such, for example, can be used. It is possible, as shown in Fig. 18(a), to fill up the chemical tank 212 with a chemical under a pressurized condition or to hold a flexible bag filled up with the chemical so as to be pressurized from the outside to open and close an intermediate point of the chemical supply tube 214 by means of a valve mechanism 217. In this case, the valve mechanism 217 is provided on the chemical diffusion apparatus side.

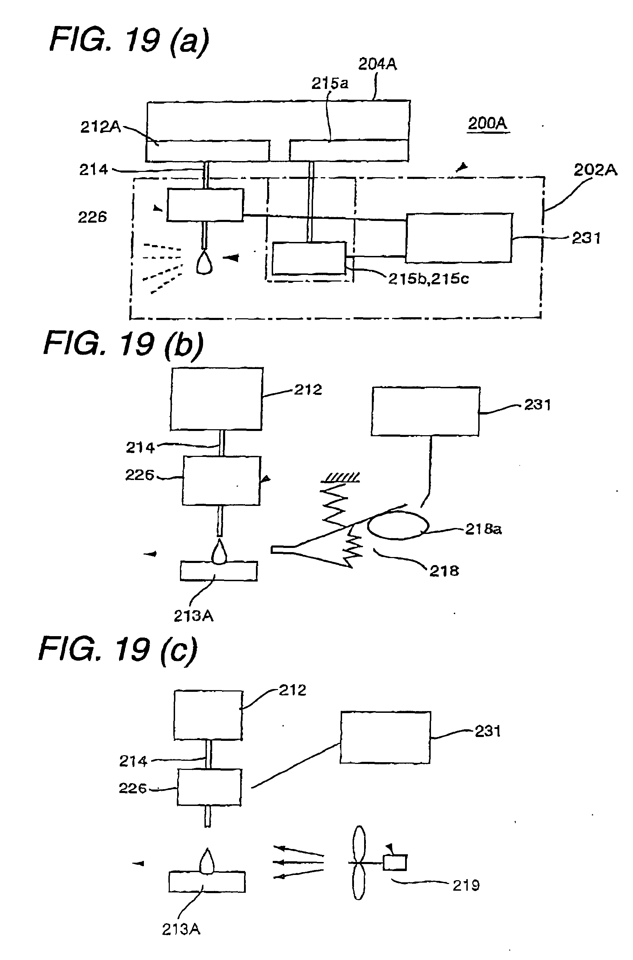

On the other hand, Fig. 19 (a) is a conceptual view of the chemical diffusion apparatus 200A of Embodiment 9. For the diffusing means, one having a structure of driving a blast mechanism 218, such as a bellows, by means of a driving mechanism 218a, such as a cam as shown in Fig. 19 (b) other than the means for natural diffusion by means of a chemical diffusion part formed from a porous material or a fibriform material or the means for diffusion by blowing the pressurized gas from a spray can, can be used. A blast fan 219 may be used instead of the above as shown in Fig. 19(c).

The invention is not limited to the embodiments described above and may be modified in various points.

For example, in Fig. 2 showing Embodiment 2, described is an example in which the chemical diffusion apparatus 20 includes chemicals having different constituents in the first tank 21, the second tank 22 and the third tank 23. One of the tanks, however, may be filled up with a diluting agent for diluting the chemical and the chemical diffusion apparatus may be fixed. In this case, the concentration of the chemical can be changed. Accordingly, it is possible to provide the optimum chemical in accordance with an ambient temperature and a subj ect insect.

Further, surfactant or the like may be filled up instead of the diluting agent or in another tank. It is also possible to discharge the respective chemicals at the optimum rate.

### Industrial applicability

As described above, in the chemical diffusion apparatus in accordance with the invention, it is arranged that a chemical be discharged from a tank filled up with the chemical by means of a discharging means such as a pump while chemical discharge volume be controlled on the basis of an environment sensor for detecting a temperature, a time period or zone, a wind direction and the like.

Therefore, in accordance with the invention, the optimum amount of chemical can be diffused at the necessary time in accordance with weather or the like, differently from a conventional method of naturally diffusing a chemical. This allows unnecessary chemical consumption to be kept down, and thereby, the maximum chemical diffusion effect to be achieved with the least chemical consumption amount. Especially, the apparatus according to the invention is suitable for diffusing a very small amount of attractant such as an insect sex pheromone for the purpose of communication disturbance of noxious insects.

Further, in accordance with the invention, a chemical cartridge is used as a chemical supply source while the chemical cartridge is arranged to carry a control parameter relating to chemical diffusion, a battery power source as a driving power source of a chemical diffusion apparatus, a drive controlling program of the chemical diffusion apparatus and such. Therefore, according to the invention, changing a chemical cartridge allows a chemical to be diffused under a driving condition suitable for diffusion of the chemical reserved in the chemical cartridge. This enables a change of a setting condition or the like of a chemical diffusion unit, which is caused by a change of a chemical, to be unnecessary or extremely easy. Moreover, the chemical diffusion unit can be standardized with the minimum required structure. Additionally, a battery changing operation due to running out of a battery of the chemical diffusion unit can be made unnecessary.

In addition to the above, the purchaser information is written in a chemical cartridge mounted to the chemical diffusion unit to construct a database of purchasers on the basis of the purchaser information written in the chemical cartridge collected after use. Using the database allows meticulous service to the purchasers to be offered.

On the other hand, according to the invention, a chemical cartridge is used as a chemical supply source while a diffusing means as well as a chemical reservoir part is mounted to the chemical cartridge. Accordingly, the diffusing means can be changed together with the chemical cartridge even in the case that the diffusing means easily becomes dirty, and thereby, maintenance such as cleaning of the diffusing means is basically unnecessary.

Further, a chemical supply tube for supplying the diffusing means with a chemical from the chemical reservoir part is mounted to the chemical cartridge. Accordingly, only changing the chemical cartridge is enough when the chemical is changed in kind, and therefore, cleaning of the chemical diffusion unit is not necessary. This allows a chemical to be easily changed in kind.

Moreover, a chemical reservoir part and a pressurized gas reservoir part are individually mounted to a chemical cartridge in the case that a spray type diffusing means is included. Accordingly, it is not necessary to check affinity whether or not the chemical and the pressurized gas can be mixed for a reserve, differently from the case of filling up a same spray can with the chemical and the pressurized gas, so that the spray type diffusing means can be used for a large number of chemicals. In addition, a used spray can used as the pressurized gas reservoir part can be easily reused since the used pressurized gas reservoir part is reusable by simply filling up the pressurized gas again without cleaning.

## Claims

1. A chemical diffusion apparatus comprising:
at least one chemical tank for reserving a chemical such as an insect sex pheromone;
a discharging means for discharging the chemical from the chemical tank;
a diffusing means for diffusing the chemical discharged from the discharging means; and
a controlling means for controlling a diffusing operation of the discharging means, wherein
the controlling means carries out drive control by varying diffusion timing and/or discharge volume of the diffusing means on the basis of hours of sunlight or sunset time, which are varied in accordance with a season, latitude and longitude and control of an artificial weather controlling apparatus.

2. The chemical diffusion apparatus according to Claim 1 further comprising:
an environment parameter detecting means for detecting at least one environment parameter of a temperature, humidity, illumination, a wind direction, a wind velocity and a chemical concentration, wherein
the controlling means controls drive of the discharging means on the basis of a detection result of the environment parameter detecting means.

3. The chemical diffusion apparatus according to Claim 1 or 2 comprising:
a time detecting means, wherein
the controlling means controls drive of the discharging means on the basis of the time detected by the time detecting means.

4. The chemical diffusion apparatus according to any one of Claims 1 to 3, wherein
the parameter memorizing means includes a self-information memorizing means for holding at least information relating to a place of installation and
the controlling means controls drive of the discharging means on the basis of the information held in the self-information memorizing means.

5. The chemical diffusion apparatus according to any one of Claims 1 to 4, wherein
the chemical tank includes plural tanks reserving respective chemical constituents included in the chemical and
the respective chemical constituents discharged from the respective tanks by means of the discharging means are mixed to form the chemical.

6. The chemical diffusion apparatus according to any one of Claims 1 to 4, wherein
the chemical tank includes plural tanks reserving different chemicals and
the discharging means can discharge the chemicals from the respective tanks individually or simultaneously.

7. The chemical diffusion apparatus according to any one of Claims 1 to 6, wherein
the chemical tank is formed from a material capable of cutting off ultraviolet rays and/or oxygen.

8. The chemical diffusion apparatus according to any one of Claims 1 to 7, wherein
the discharging means is a pump variable in discharge volume of a chemical.

9. The chemical diffusion apparatus according to any one of Claims 1 to 7, wherein
the discharging means includes a chemical pressure chamber supplied with the chemical from the chemical tank, a chemical discharge nozzle communicating with the chemical pressure chamber and an actuator for generating variations in pressure of the chemical in the chemical pressure chamber to discharge liquid drops of the chemical from the chemical discharge nozzle.

10. The chemical diffusion apparatus according to anyone of Claims 1 to 9, wherein
the diffusing means includes an evaporating dish for evaporating the chemical or a chemical holding member formed from a porous material or a fibriform material for holding the chemical so as to be capable of natural diffusion.

11. The chemical diffusion apparatus according to any one of Claims 1 to 9, wherein
the diffusing means includes a chemical holding member formed from a porous material or a fibriform material for holding the chemical so as to be capable of natural diffusion and a carrying mechanism for circulating the chemical holding member along a predetermined carrying path.

12. The chemical diffusion apparatus according to any one of Claims 1 to 11 comprising:
a chemical cartridge used as the chemical tank; and
a chemical diffusion unit using the chemical cartridge as a chemical supply source, wherein
the chemical diffusion unit includes a cartridge mounting part for changeably mounting the chemical cartridge, the discharging means, the diffusing means, the environment parameter detecting means and the controlling means.

13. The chemical diffusion apparatus according to Claim 1 comprising:
a chemical cartridge used as the chemical tank; and
a chemical diffusion unit using the chemical cartridge as a chemical supply source, wherein
the chemical cartridge includes a chemical reservoir part for reserving the chemical and a cartridge side memorizing means for memorizing at least one controlling parameter relating to the chemical diffusing operation,
the chemical diffusion unit includes a cartridge mounting part for changeably mounting the chemical cartridge, the discharging means, the diffusing means, the environment parameter detecting means and the controlling means, and
the controlling means controls drive of the discharging means on the basis of the controlling parameter memorized in the cartridge side memorizing means of the chemical cartridge mounted to the cartridge mounting part.

14. The chemical diffusion apparatus according to Claim 13, wherein
the chemical diffusion unit includes a unit side memorizing means for memorizing at least one controlling parameter relating to the chemical diffusing operation, the controlling parameter being different from the controlling parameter held in the chemical cartridge and
the controlling means controls drive of the discharging means on the basis of the controlling parameter of the chemical cartridge and the controlling parameter of the chemical diffusion unit.

15. The chemical diffusion apparatus according to Claim 13 or 14, wherein
the controlling parameter is at least one of the following (a) to (f):
(a) the kind of the chemical;
(b) the volume and the remaining amount of the chemical;
(c) the kind of a noxious insect subject to extermination by means of the chemical;
(d) the optimum condition for discharging the chemical by means of the discharging means;
(e) location information of the chemical diffusion unit; and
(f) the chemical discharge power of the discharging means.

16. The chemical diffusion apparatus according to Claim 15, wherein
the controlling parameter held in the chemical cartridge includes at least the optimum condition of discharging the chemical and
the controlling parameter held in the chemical diffusion unit includes at least the location information of the chemical diffusion apparatus.

17. The chemical diffusion apparatus according to any one of Claims 13 to 16, wherein
the controlling means of the chemical diffusion unit is formed around a computer,
the cartridge side memorizing means memorizes a drive controlling program of the controlling means and
the controlling means reads the drive controlling program when the chemical cartridge is mounted, and then, executes the drive controlling program to perform an operation of diffusing the chemical.

18. The chemical diffusion apparatus according to Claim 17, wherein
the chemical diffusion unit includes a plurality of the cartridge mounting parts and
the controlling means reads the drive controlling program from the chemical cartridge having the first priority determined on the basis of the order of priority of the chemical cartridge, the order of priority of the cartridge mounting part of the chemical diffusion unit or the order of mounting the chemical cartridge.

19. The chemical diffusion apparatus according to any one of Claims 13 to 18, wherein
the chemical cartridge includes a battery power source and
the chemical diffusion unit is supplied with driving power from the battery power source of the chemical cartridge mounted to the cartridge mounting part.

20. The chemical diffusion apparatus according to any one of Claims 13 to 19, wherein
the unit side memorizing means of the chemical diffusion unit memorizes unit identification information for identifying the chemical diffusion unit and information of customer purchasing the chemical diffusion unit and
the controlling means reads the unit identification information and the purchaser information to memorize the same in the cartridge side memorizing means when the chemical cartridge is mounted.

21. The chemical diffusion apparatus according to Claim 20 including as the cartridge side memorizing means:
a writing apparatus for writing at least one of the control parameters, the drive controlling program or the purchaser information; and
a database construction apparatus for reading the unit identification information and the purchaser information, which are held in the used chemical cartridge, to construct a database relating to the purchasers.

22. The chemical diffusion apparatus according to Claim 1 comprising:
a chemical cartridge used as the chemical tank; and
a chemical diffusion unit using the chemical cartridge as a chemical supply source, wherein
the chemical cartridge includes a chemical reservoir part for reserving the chemical, the diffusing means and/or the chemical supply tube for connecting the chemical reservoir part with the diffusion means and
the chemical diffusion unit includes at least a cartridge mounting part for changeably mounting the chemical cartridge, the environment parameter detecting means and the controlling means.

23. The chemical diffusion apparatus according to Claim 22, wherein
the discharging means includes a chemical supply pump for supplying the diffusing means with the chemical in the chemical reservoir part,
the chemical supply pump is a tube pump including the chemical supply tube and a driving part for pressuring the chemical supply tube to send out the chemical in the chemical tube and
the chemical supply tube is mounted to the chemical cartridge while the driving part is mounted to the chemical diffusion unit.

24. The chemical diffusion apparatus according to Claim 22, wherein
the discharging means includes a pressurization mechanism for pressurizing the chemical reserved in the chemical reservoir part and a valve mechanism for opening and closing the chemical supply tube and
the chemical diffusion unit includes the pressurization mechanism and the valve mechanism.

25. The chemical diffusion apparatus according to any one of Claims 22 to 24, wherein
the diffusion means is a chemical holding member formed from a porous material, a fibriform material or the like for holding the chemical discharged from the discharging means so as to be capable of natural diffusion.

26. The chemical diffusion apparatus according to Claim 22, wherein
the chemical cartridge includes a chemical spray mechanism for diffusing a chemical in the air by means of pressurized gas and
the chemical spray mechanism functions as the discharging means and the diffusing means.

27. The chemical diffusion apparatus according to Claim 26, wherein
the chemical spray mechanism includes a pressurized gas reservoir part and the pressurized gas nozzle and
the chemical diffusing unit includes an opening/closing mechanism for opening and closing the pressurized gas nozzle.

28. The chemical diffusion apparatus according to any one of Claims 22 to 27, wherein
at least one of the chemical reservoir part, the diffusing means, the chemical supply tube and the pressurized gas reservoir part is changeably mounted to the chemical cartridge.

29. A mobile chemical diffusion system comprising:
a chemical diffusion apparatus;
a traveling mechanism to which the chemical diffusion apparatus is mounted; and
a travel controlling means for controlling drive of the traveling mechanism, wherein
the chemical diffusion apparatus is the chemical diffusion apparatus according to any one of Claims 1 to 28.

30. A floating chemical diffusion system comprising:
a chemical spray apparatus; and
a balloon on which the chemical spray apparatus is hung, wherein
the chemical diffusion apparatus is the chemical diffusion apparatus according to any one of Claims 1 to 28.

31. A chemical diffusion system comprising:
plural chemical diffusion apparatuses provided in different places; and
a center controlling means for controlling respective chemical discharging operations of the chemical diffusion apparatuses, wherein
the chemical diffusion apparatus is the chemical diffusion apparatus according to any one of Claims 1 to 28 and
the center controlling means controls discharge and/or discharge volume of the chemicals in the respective chemical diffusion apparatuses on the basis of a place of installing the respective diffusion apparatuses and a detection result by the environment parameter detecting means of the respective chemical diffusion apparatuses.

32. The chemical cartridge according to any one of Claims 12 to 28.

33. The chemical diffusion unit according to any one of Claims 12 to 28.
